(19) Europäisches Patentamt
European Patent Office
Office européen des brevets

(11) EP 3 668 652 B1

(12) **FASCICULE DE BREVET EUROPEEN**

(45) Date de publication et mention de la délivrance du brevet:
**06.09.2023 Bulletin 2023/36**

(21) Numéro de dépôt: **18762584.3**

(22) Date de dépôt: **08.08.2018**

(51) Classification Internationale des Brevets (IPC):
***B03D 1/01*** *(2006.01)* ***A61K 8/85*** *(2006.01)*
***C08G 63/685*** *(2006.01)* ***D06M 15/507*** *(2006.01)*

(52) Classification Coopérative des Brevets (CPC):
**C08G 63/6854; A61K 8/85; B03D 1/011; C08G 63/6856; D06M 15/507**

(86) Numéro de dépôt international:
**PCT/FR2018/052034**

(87) Numéro de publication internationale:
**WO 2019/034817 (21.02.2019 Gazette 2019/08)**

(54) **POLYESTERAMINES ET POLYESTERQUATS**

POLYESTERAMINE UND POLYESTERQUATS

POLYESTERAMINES AND POLYESTER QUATS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats de validation désignés:
**MA**

(30) Priorité: **16.08.2017 FR 1757704**

(43) Date de publication de la demande:
**24.06.2020 Bulletin 2020/26**

(73) Titulaire: **Arkema France**
**92700 Colombes (FR)**

(72) Inventeurs:
- **JORDA, Eric**
**69005 Lyon (FR)**
- **BALOCHE, Alain**
**62880 Annay (FR)**
- **BARRETO, Gilles**
**69510 Messimy (FR)**
- **GILLET, Jean-Philippe**
**69530 Brignais (FR)**

(56) Documents cités:
**EP-A2- 0 035 263** **EP-A2- 2 576 072**
**JP-A- H08 291 281** **US-A- 4 606 916**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Processed by Luminess, 75001 PARIS (FR)

## Description

La présente invention concerne des composés ayant des structures de polyesteramine et polyesterquat. De telles structures sont nouvelles et des composés avec une telle structure peuvent être utilisés dans de nombreux domaines d'application et, par exemple, en tant qu'agents tensioactifs, en tant qu'inhibiteurs de corrosion et similaire.

Des alkylamines grasses et des composés d'ammonium quaternaire sont des substances chimiques particulièrement utiles employées dans une grande variété d'industries. Parmi toutes les applications possibles, il peut être mentionné leur utilisation en tant que biocides, produits de soin capillaire, assouplissants de textile ou additifs antistatiques pour des matières plastiques. Leurs fortes propriétés d'adsorption sur des surfaces minérales ou métalliques ou d'autres particules solides, les rendent particulièrement intéressants en tant qu'agents mouillants, inhibiteurs de corrosion, collecteurs pour l'enrichissement de minerai, additif d'attaque acide de roche de phosphate, engrais et additif de traitement de surface ou interne de sels minéraux, antiagglomérants pour des hydrates, modificateurs d'argile, additifs de lubrification ou promoteurs d'adhérence. Ils peuvent également présenter des propriétés viscosifiantes, émulsifiantes ou stabilisantes utiles pour l'industrie du pétrole et du gaz (agents désémulsifiants, tensioactifs pour une récupération de pétrole augmentée, tensioactifs pour fluides de fracture, tensioactifs pour acidifier des fluides, tensioactifs pour amélioration de l'injectivité d'eau produite, tensioactifs pour boues de forage), l'industrie d'émulsion de bitume ou détergence.

Ces alkylamines grasses et composés d'ammonium quaternaire sont bien connus dans l'industrie et la recherche est continue pour trouver de nouveaux produits. Au cours des 30 dernières années, une autre famille de composés contenant de l'azote de poids moléculaire plus lourd a été décrite dans la littérature. Ces composés sont des amines et un ammonium quaternaire avec une structure polymère ou oligomère.

Par exemple, le brevet EP0035263 décrit un produit de réaction entre un acide dicarboxylique et une amine grasse éthoxylée, ledit produit étant utilisé en tant qu'assouplissant de textile. Dans le brevet EP0144975, le même produit de réaction peut être quaternarisé et utilisé en tant que conditionneur capillaire.

La demande WO2008/089906 décrit un produit de réaction d'un acide gras avec un diacide et une (alkyl)polyéthanolamine, ledit produit de réaction étant ensuite quaternarisé et utilisé en tant que collecteur pour la flottation de minerais non-sulfure.

De manière similaire, JP08-291281 décrit des produits de réaction d'alkylamines en $C_1$-$C_{22}$ alcoxylées avec des diacides (ou des anhydrides) suivie d'une réaction de quaternarisation, lesdits produits étant utilisés en tant qu'agent antistatique dans des résines composites, en particulier pour l'industrie automobile.

WO 2011/147855 décrit en outre un produit de réaction d'un alcool gras avec un diacide et une (alkyl)polyéthanolamine, facultativement suivie d'une réaction de quaternarisation partielle ou totale, un tel produit étant typiquement utilisé pour la flottation de silicates. L'alcool gras peut être remplacé par une amine grasse alkylée comportant une seule chaîne alcoxylée avec une seule fonction -OH de terminaison.

Par conséquent, il existe encore un besoin de nouveaux composés oligomères ou polymères cationiques pour utilisation dans les domaines d'application énumérés ci-dessus, ledit composé étant plus facilement préparé, plus stable, moins coûteux à synthétiser et présentant en outre de meilleures propriétés environnementales en termes de toxicité et de biodégradabilité, et ayant des performances techniques améliorées.

Par conséquent, la présente invention concerne un composé pouvant être obtenu par la condensation par estérification de :

A/ une amine grasse alcoxylée de formule (I), ou du produit de quaternarisation partielle ou totale de ladite amine grasse alcoxylée de formule (I) :

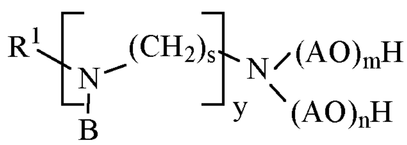

(I)

dans laquelle :

- $R^1$ est choisi parmi un groupe hydrocarbyle ayant 8 à 24 atomes de carbone, de préférence 10 à 24, plus préférablement 12 à 24 atomes de carbone, et un groupe de formule $R^4$-O-(A'O)$_w$-T-, dans laquelle $R^4$ est un groupe hydrocarbyle ayant 8 à 24 atomes de carbone, de préférence 12 à 24 atomes de carbone, w représente un entier dans la plage de 0 à 20, de préférence de 0 à 10, plus préférablement de 0 à 6, et encore plus préférablement de 0 à 4, A'O est un groupe alkylénoxy contenant 2 à 4 atomes de carbone, de préférence 2 ou 3 atomes de carbone, plus préférablement 2 atomes de carbone ; T est alkylène avec 1 à 6 atomes de

carbone, de préférence 1 à 4 atomes de carbone, de manière préférée entre toutes 2 ou 3 atomes de carbone,

- AO est un groupe alkylénoxy contenant 2 à 4 atomes de carbone, de préférence 2 ou 3 atomes de carbone, plus préférablement 2 atomes de carbone,
- B est choisi parmi un groupe alkyle en $C_1$-$C_4$, aryle ou arylalkyle group (par exemple phényle, phénylalkyle, tel que benzyle),
- m représente un entier compris entre 1 et 20, de préférence entre 1 et 10, plus préférablement entre 1 et 6, et encore plus préférablement entre 1 et 4, limites incluses,
- n représente un entier compris entre 1 et 20, de préférence entre 1 et 10, plus préférablement entre 1 et 6, et encore plus préférablement entre 1 et 4, limites incluses,
- s est 1, 2 ou 3, de préférence 2 ou 3, et
- y est un entier de 0 à 5, de préférence de 0 à 3, plus préférablement y est 0 ou 1, encore plus préférablement y est 0,

B/ avec un acide dicarboxylique, ou un dérivé de celui-ci, de formule (II) :

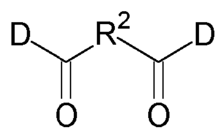

(II),

dans laquelle

- D est choisi parmi -F, -Cl, Br et -$OR^3$, où $R^3$ est hydrogène ou un groupe alkyle en $C_1$-$C_4$,
- $R^2$ est choisi dans le groupe constitué de :
    - une liaison directe,
    - une chaîne hydrocarbonée linéaire ou ramifiée, saturée ou insaturée en $C_1$-$C_{20}$ facultativement substituée par un ou plusieurs groupe(s) -OH, de préférence un radical alkylène de formule $-(CH_2)_z-$, dans laquelle z est un entier de 1 à 20, de préférence de 1 à 10, de préférence de 2 à 6, et de manière préférée entre toutes 4, un radical alkylène substitué, ledit radical alkylène étant substitué par 1 ou 2 groupes -OH, un radical alcénylène ayant de 1 à 20, de préférence de 1 à 10 atomes de carbone, un radical alcénylène substitué, ledit radical alcénylène étant substitué par 1 ou 2 groupes méthyle et/ou méthylène,
    - un groupe cycloalkylène,
    - cycloalcénylène et
    - arylène

C/ avec un dérivé de (alkyl)alcanolamine de formule (III) ou du produit de quaternarisation partielle ou totale dudit dérivé de (alkyl)alcanolamine de formule (III) :

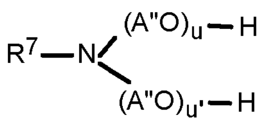

(III)

dans laquelle :

- A"O représente un groupe alkylénoxy contenant de 2 à 4 atomes de carbone, de préférence 2 ou 3 atomes de carbone, plus préférablement 2 atomes de carbone,
- u représente un entier compris entre 1 et 20, de préférence entre 1 et 10, plus préférablement entre 1 et 6, et encore plus préférablement entre 1 et 4, limites incluses,
- u' représente un entier compris entre 1 et 20, de préférence entre 1 et 10, plus préférablement entre 1 et 6, et encore plus préférablement entre 1 et 4, limites incluses,
- $R^7$ est choisi parmi un groupe hydrocarbyle ayant 1 à 7, de préférence 1 à 6 atomes de carbone, plus préférablement 1 à 4 atomes de carbone, un groupe aryle ou arylalkyle (par exemple, un groupe phényle ou naphtyle), un groupe de formule $H-(OA")_v-$ (dans laquelle v représente un entier compris entre 1 et 20, de préférence entre 1 et 10, plus préférablement entre 1 et 6, et encore plus préférablement entre 1 et 4, limites incluses), $HO(CH_2)_q-$ et un groupe de formule (IV)

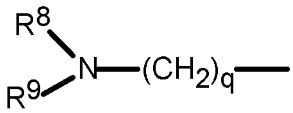

(IV),

- dans laquelle $R^8$ et $R^9$, identiques ou différents, sont choisis parmi un groupe hydrocarbyle ayant 1 à 6 atomes de carbone, de préférence 1 à 4 atomes de carbone et q est un entier de 1 à 10, de préférence de 2 à 6, limites incluses, et de manière préférée entre toutes q est 2 ou 3,
- ou $R^8$ et $R^9$, conjointement avec l'atome d'azote auquel ils sont liés forment un cycle de 5, 6 ou 7 atomes, comportant facultativement un ou plusieurs hétéroatome(s) choisi(s) parmi l'oxygène, l'azote ou le soufre.

Dans la présente description, « hydrocarbyle » désigne une chaîne hydrocarbyle linéaire ou ramifié, saturé ou insaturé.

Dans un mode de réalisation préféré, des composés de l'invention peuvent être obtenus par la condensation par estérification telle que définie ci-dessus, dans laquelle, dans l'amine grasse de formule (I), $R^1$ comporte 8, ou plus de 8, atomes de carbone, typiquement de 8 à 24 atomes de carbone, de préférence de 10 à 24, plus préférablement de 12 à 24 atomes de carbone, limites incluses, et dans le dérivé de (alkyl)alcanolamine de formule (III), $R^7$ comporte 6 atomes de carbone ou moins, typiquement de 1 à 6 atomes de carbone, plus préférablement 1 à 4 atomes de carbone, limites incluses.

Dans un mode de réalisation encore plus préféré, des composés de l'invention peuvent être obtenus par la condensation par estérification telle que définie ci-dessus, dans laquelle, dans l'amine grasse de formule (I) et dans le dérivé de (alkyl)alcanolamine de formule (III), $R^1$ et $R^7$ sont tels que la différence de nombre d'atomes de carbone qu'ils comportent est supérieur à 2, typiquement de 2 à 23, de préférence 5 à 23, plus préférablement 10 à 23, limites incluses.

Il est entendu que les acides dicarboxyliques ou dérivés de ceux-ci de formule (II) comprennent en outre leurs formes anhydrides correspondantes. Il est en outre entendu que lorsque la chaîne alkylénoxy contient plus d'un groupe alkylénoxy, les groupes alkylénoxy peuvent être identiques ou différents. De manière similaire, lorsque y est supérieur à un, les motifs de répétition peuvent être identiques ou différents.

La présente invention concerne en outre le composé de la présente invention (pouvant être obtenu par réaction entre l'amine grasse alcoxylée de formule (I), l'acide dicarboxylique ou un dérivé de celui-ci de formule (II), et le dérivé de (alkyl)alcanolamine de formule (III)), après réaction supplémentaire dans laquelle une partie ou la totalité des atomes d'azote sont quaternarisés par réaction avec un réactif de formule $R^5X$, dans laquelle $R^5$ est choisi parmi un groupe hydrocarbyle en $C_1$-$C_6$, de préférence un groupe alkyle en $C_1$-$C_4$, phényle et phénylalkyle, tel que benzyle, et X est un groupe partant quelconque connu dans l'art et, de préférence, X est généralement choisi parmi des halogènes, des sulfates, des carbonates, et similaire.

La formule (1) ci-dessous est une représentation possible des composés de la présente invention, comme décrit ci-dessus, pouvant être obtenus par la condensation par estérification de l'amine grasse alcoxylée de formule (I) (comprenant son produit de quaternarisation partielle ou totale), avec l'acide dicarboxylique, ou un dérivé de celui-ci, de formule (II), et avec un dérivé de (alkyl)alcanolamine de formule (III) (comprenant son produit de quaternarisation partielle ou totale).

Par conséquent, et dans un deuxième aspect, la présente invention concerne un composé de formule générale (1) :

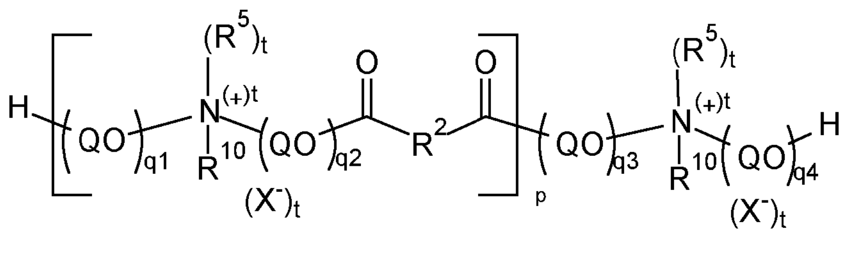

(1)

dans laquelle :

- $R^2$, $R^5$ et X sont tels que définis ci-dessus,
- t est 0 ou 1
- p est un entier dans la plage de 1 à 15, de préférence de 1 à 10, plus préférablement de 1 à 5, limites incluses,
- QO représente un groupe alkylénoxy contenant de 2 à 4 atomes de carbone, de préférence 2 ou 3 atomes de

carbone, plus préférablement 2 atomes de carbone, sachant que tous les Q présents dans le composé de formule (1) peuvent être identiques ou différents,

- $q_1$, $q_2$, $q_3$, $q_4$, identiques ou différents les uns des autres, représentent chacun un entier compris entre 1 et 20, de préférence entre 1 et 10, plus préférablement entre 1 et 6, et encore plus préférablement entre 1 et 4, limites incluses,
- chaque groupe $R^{10}$, indépendamment des autres, représente $R^7$ comme précédemment défini ou un groupe $R^1$-$(G)_y$- dans lequel $R^1$ et y sont tels que précédemment définis et G représente un groupe de formule (IV) :

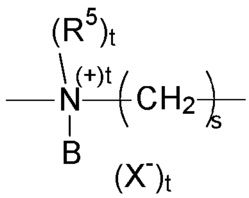
(IV)

dans laquelle B, $R^5$, X, t et s sont tels que définis ci-dessus, le groupe $(CH_2)_s$ est un espaceur entre les deux atomes d'azote auxquels il est lié,
sachant qu'au moins un des groupes $R^{10}$ représente $R^7$, et au moins un autre des groupes $R^{10}$ représente $R^1$-$(G)_y$- et chaque t est indépendant des autres.

Dans un mode de réalisation particulier, l'amine grasse alcoxylée de formule (I) est de formule (IA) :

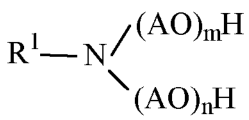
(IA)

qui est l'amine grasse alcoxylée de formule (I) dans laquelle y représente 0, et $R^1$, AO, m et n sont tels que définis ci-dessus,
ainsi que ses dérivés partiellement ou totalement quaternarisés correspondants.

Dans un autre mode de réalisation particulier, le dérivé de (alkyl)alcanolamine de formule (III) est de formule (IIIA) :

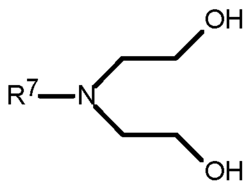
(IIIA)

qui est la (alkyl)alcanolamine de formule (III) dans laquelle u et u' représentent chacun 1, A"O est éthylénoxy et $R^7$ est tel que défini ci-dessus. Dans le composé de formule (IIIA) ci-dessus, $R^7$ est de préférence un groupe hydrocarbyle ayant 1 à 4 atomes de carbone. Le composé de formule (IIIA) ci-dessus couvre en outre ses dérivés partiellement ou totalement quaternarisés correspondants.

Le dérivé d'acide dicarboxylique dérivé de formule générale (II) décrit peut être un acide dicarboxylique ou dérivé d'acide dicarboxylique ou anhydride quelconque connu de l'homme du métier, et typiquement un acide dicarboxylique, un halogénure, par exemple un chlorure, d'acide dicarboxylique, un diester d'un acide dicarboxylique, ou un anhydride cyclique d'un acide dicarboxylique. Les dérivés les plus adaptés sont les acide dicarboxyliques et leurs anhydrides cycliques correspondants.

Des exemples illustratifs de dérivés d'acide dicarboxylique de formule générale (II) comprennent l'acide oxalique, l'acide malonique, l'acide succinique, l'acide glutarique, l'acide glutaconique, l'acide adipique, l'acide muconique, l'acide pimélique, l'acide phtalique et ses isomères, l'acide tétrahydrophtalique, l'acide malique, l'acide maléique, l'acide fumarique, l'acide subérique, l'acide mésaconique, l'acide sébacique, l'acide azélaïque, l'acide tartrique, l'acide itaconique, l'acide glutinique, l'acide citraconique, l'acide brassylique, l'acide dodécanedioïque, l'acide traumatique, l'acide thapsique, leurs chlorures d'acide correspondants, leurs esters méthyliques ou éthyliques correspondants, et leurs anhydrides cycliques correspondants, ainsi que des mélanges de ceux-ci.

Des dérivés d'acide dicarboxylique de formule générale (II) préférés sont choisis parmi mentionné ci-dessus l'acide oxalique, l'acide malonique, l'acide succinique, l'acide glutarique, l'acide adipique, l'acide pimélique, l'acide phtalique et ses isomères, l'acide tétrahydrophtalique, l'acide malique, l'acide tartrique, l'acide itaconique, leurs chlorures d'acide correspondants, leurs esters méthyliques ou éthyliques correspondants, et leurs anhydrides cycliques correspondants, ainsi que des mélanges de ceux-ci.

Des amines grasses alcoxylées de formule (I) sont disponibles ou peuvent être préparées selon un procédé connu dans la littérature, et peuvent être, par exemple, aisément préparés par alcoxylation d'amines grasses de formule (a) :

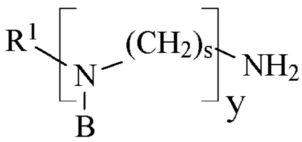

(a)

dans laquelle $R^1$, B, s et y sont tels que définis ci-dessus.

Des exemples illustratifs d'amines grasses adaptées selon la formule (a) pour utilisation en tant que matériaux de départ pour la préparation d'amines grasses alcoxylées de formule (I) comprennent, mais ne sont pas limités à, des amines grasses de formule (a1) dans laquelle y représente 0 et des amines grasses de formule (a2) dans laquelle y représente 1, s représente 3 et B représente méthyle :

$R^1\text{-}NH_2$ (a1)

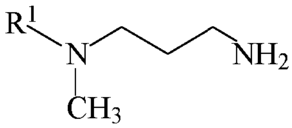

(a2)

dans laquelle $R^1$ est tel que défini ci-dessus.

Des exemples particuliers d'amines de formule (a1) sont des amines de formule (a3) :

$R^4\text{-}O\text{-}T\text{-}NH_2$ (a3),

dans laquelle $R^4$, et T sont tels que définis ci-dessus et w est 0.

Des exemples plus spécifiques des amines de formule (a) mentionnées ci-dessus comprennent, mais ne sont pas limités à, la 2-éthylhexylamine, la 2-propylheptylamine, la n-octylamine, la n-décylamine, la n-dodécylamine, la (coco-alkyl)amine, la (huile de palme-alkyl)amine, la n-tétradécylamine, la n-hexadécylamine, la n-octadécylamine, l'oléylamine, la (suif-alkyl)amine, la (suif hydrogéné-alkyl)amine, la (colza-alkyl)amine, la (soja-alkyl)amine, l'érucylamine, la N-(n-décyl)-N-méthyl-triméthylènediamine, la N-(n-dodécyl)-N-méthyl-triméthylènediamine, la N-(coco-alkyl)-N-méthyl-triméthylènediamine, la N-(colza-alkyl)-N-méthyl-trimé-thylènediamine, la N (soja-alkyl)-N-méthyl-triméthylènediamine, la N-(suif-alkyl)-N-méthyl-triméthylènediamine, la N-(suif hydrogéné-alkyl)-N-méthyl-triméthylène-diamine, la N-érucyl-N-méthyltriméthylènediamine et l'isotridécyloxypropylamine, ainsi que des mélanges de celles-ci.

Selon un mode de réalisation de l'invention, les amines mentionnées ci-dessus sont des amines grasses obtenues à partir d'huile ou d'acides naturels (végétaux ou animaux) et des mélanges de ceux-ci, par exemple des acides gras de noix de coco, des acides gras de suif, des huiles de colza, des huiles de soja et des huiles de palme.

Ces amines grasses sont ensuite typiquement alcoxylées avec 2 à 40, de préférence 2 à 20, plus préférablement 2 à 12 et encore plus préférablement 2 à 8 EO (motifs d'oxyde d'éthylène), et/ou 2 à 40, de préférence 2 à 20, plus préférablement 2 à 12 et encore plus préférablement 2 à 8 PO (motifs d'oxyde de propylène), et/ou 2 à 40, de préférence 2 à 20, plus préférablement 2 à 12 et encore plus préférablement 2 à 8 BO (motifs d'oxyde de butylène). Des blocs avec EO sont généralement ajoutés en premier et PO et/ou BO en dernier, ou des blocs avec PO et/ou BO ajoutés en premier et EO en dernier, ou avec des mélanges d'EO et PO et/ou BO pour produire des produits alcoxylés de façon aléatoire de formule générale (I). L'alcoxylation peut être effectuée par un procédé adapté quelconque connu dans l'art en utilisant, par exemple, un catalyseur alcalin, tel que l'hydroxyde de potassium (KOH), ou un catalyseur acide ou même sans catalyseur.

Des exemples de produits de formule (I) commercialisés comprennent Noramox® SD20, Noramox® SD15,

Noramox® S11, Noramox® S5, Noramox® S7, Noramox® S2, Noramox® SH2, Noramox® O2, Noramox® O5, Noramox® C2, Noramox® C5, Noramox® C15. Tous ces produits commercialisés sont disponibles auprès de CECA S.A. D'autres exemples de produits de formule (I) commercialisés comprennent Tomamine® E-17-5 et Tomamine® E-T-2, commercialisés par Air Products.

Des dérivés de (alkyl)alcanolamine de formule (III) sont disponibles ou peuvent être préparés selon un procédé décrit dans la littérature. Des exemples illustratifs de dérivés de (alkyl)alcanolamines de formule (III) adaptés comprennent, mais ne sont pas limités à, la triéthanolamine, la méthyldiéthanolamine, l'éthyldi-éthanolamine, la propyldiéthanolamine, la butyldiéthanolamine, l'isobutyldiéthanol-amine, la pentyldiéthanolamine, la phényldiéthanolamine, l'hexyldiéthanolamine, l'heptyldiéthanolamine, ainsi que leurs produits d'alcoxylation correspondants.

D'autres exemples d'amines adaptées en tant que matériau de départ pour la préparation de dérivés alcoxylés de formule (III) comprennent, sans limitation, la méthylamine, l'éthylamine, les propylamines, les butylamines, les pentylamines, les hexylamines, les heptylamines, la diméthylaminoéthylamine, la diéthylamino-éthylamine, la diméthylaminopropylamine (DMAPA), la diéthylaminopropylamine (DEAPA), la dipropylaminopropylamine, la dibutylaminopropylamine (DBAPA), la 1-(3-aminopropyl)-2-pyrolidine, la 3-morpholinopropylamine, la 1-(3-aminopropyl)pipé-ridine, la 1-(3-aminopropyl)pipécoline. Certains de ces dérivés alcoxylés de formule (III) sont nouveaux et, en tant que tels, font partie de la présente invention.

Un procédé adapté pour la préparation des produits pour utilisation dans la présente invention comprend les étapes de mélange d'au moins un composé de formule (II) avec au moins un composé de formule (III) tel que défini ci-dessus et au moins un composé de formule (I) tel que défini ci-dessus, et conduite d'une réaction de condensation par estérification entre les composés dans le mélange.

Comme il apparaîtra à l'homme du métier, d'autres procédés dans lesquels des composés de formule (I), (II) et (III) réagissent séquentiellement les uns avec les autres dans différents ordres sont également adaptés. Par exemple, il est possible de conduire une réaction de condensation par estérification entre des composés de formule (I) et (II) dans un premier temps, et ensuite de conduire une autre réaction de condensation par estérification de ce produit de condensation avec un composé de formule (III) dans une étape supplémentaire. Un autre procédé adapté comprend la conduite d'une réaction de condensation par estérification entre des composés de formule (II) et (III) dans un premier temps, et ensuite de conduire une autre réaction de condensation par estérification de ce produit de condensation avec un composé de formule (I) dans une étape supplémentaire.

D'autres procédés alternatifs comprennent la conduite d'une réaction de condensation par estérification, telle que par exemple, entre :

a. un produit de réaction de condensation entre des composés de formule (II) et (III) et
b. un produit de réaction de condensation entre des composés de formule (I) et (II)
c. facultativement la conduite d'une autre réaction de condensation par estérification des composés de produit de réaction de l'étape a et l'étape b ci-dessus conjointement avec au moins un composé de formule (I) et/ou formule (II) et/ou formule (III).

Plus généralement, les composés de formule (1) de la présente invention peuvent être préparés à partir de réaction(s) de condensation par estérification dans lesquelles au moins un composé de formule (I) et au moins un composé de formule (II) et au moins un composé de formule (III) réagissent dans une ou plusieurs réaction(s) de condensation par estérification simultanées et/ou séquencées et/ou alternées.

La réaction de condensation par estérification se produisant entre les composés de formule (II) et de formule (I) et (III) est une réaction connue en tant que telle dans l'art. La réaction est de préférence conduite en présence d'un catalyseur d'estérification, tel qu'un acide de Bronstedt ou un acide de Lewis, par exemple l'acide méthanesulfonique, l'acide paratoluènesulfonique, l'acide hypophosphorique, l'acide citrique ou le trifluorure de bore ($BF_3$).

Lorsqu'un dérivé d'acide dicarboxylique de formule (II), dans lequel D est $OR^3$, est utilisé, la réaction est une transestérification qui, en variante, peut être conduite en présence d'un catalyseur alcalin. En variante, d'autres techniques conventionnelles connues de l'homme du métier peuvent être utilisées à partir d'autres dérivés des acides dicarboxyliques, par exemple à partir de leurs anhydrides ou de leurs chlorures d'acide.

Comme il apparaîtra à l'homme du métier, les différentes réactions d'estérification peuvent être conduites avec ou sans ajout de solvants. Si des solvants sont présent pendant la réaction, les solvants doivent être inertes pour l'estérification, par exemple, le toluène ou le xylène, et similaire.

La réaction de condensation par estérification entre les composants (I), (II) et (III) peut être conduite à une température quelconque dans des conditions opératoires connues et, par exemple, à une température typiquement dans la plage de 60°C à 300 °C, de préférence de 120 °C à 280°C, et généralement pendant une durée dans la plage de 1 heure à plusieurs heures, de préférence de 2 heures à 20 heures. La réaction de condensation par estérification peut être conduite à pression atmosphérique, en variante ladite réaction peut facultativement être conduite à une pression réduite, par exemple de 500 Pa à 20000 Pa.

Dans un mode de réalisation spécifique de la présente invention, le rapport molaire entre les réactifs [(I) + (III)] et (II) est de 2:1 à 1:2, de préférence 1,5:1 à 1:1,5, et de manière préférée entre toutes 1,4:1 à 1:1,4.

Selon un autre mode de réalisation spécifique de la présente invention, le rapport molaire entre les réactifs [(I) + (III)] et (II) est de 2:1 à 1:1, de préférence 2:1 à 1,2:1, et de manière préférée entre toutes 2:1 à 1,3:1.

Selon un autre mode de réalisation supplémentaire, le rapport molaire entre (I) et (III) est de 15:1 à 1:15, de préférence 10:1 à 1:10, plus préférablement 4:1 à 1:4, et de manière préférée entre toutes 2:1 à 1:2.

Lorsqu'un produit quaternaire est souhaité, le procédé de préparation peut comprendre en outre au moins une étape constituée de l'ajout d'un agent d'alkylation au produit de de condensation réaction et la conduite de ladite réaction de quaternarisation du produit de condensation, selon des techniques connues dans l'art.

En tant que tel, lorsque tous les t sont 0 dans la formule (1), le produit est un composé de polyesteramine tertiaire, et lorsque tous les t sont 1, le produit est un composé de polyester polyammonium quaternaire, résultant de la quaternarisation du composé dans lequel t est 0. Comme il apparaîtra à l'homme du métier, lorsqu'une partie des t sont 0 et une partie des t sont 1, le produit est un composé de polyesteramine partiellement quaternarisé.

Pour l'étape de réaction de quaternarisation, Les agents d'alkylation préférés sont choisis parmi des composés de formule $R^5X$. Des exemples illustratifs de tels agents d'alkylation comprennent, mais sont pas limités à, le chlorure de méthyle, le bromure de méthyle, l'iodure de méthyle, le sulfate de diméthyle, le sulfate de diéthyle, le carbonate de diméthyle et le chlorure de benzyle, les agents d'alkylation préférés entre tous étant le chlorure de méthyle, le sulfate de diméthyle, le sulfate de diéthyle ou le chlorure de benzyle et des mélanges de ceux-ci, de préférence le chlorure de méthyle et/ou le sulfate de diméthyle.

Selon une variante, la quaternarisation peut être effectuée sur l'amine grasse de formule (I) et/ou sur le dérivé de (alkyl)alcanolamine de formule (III) avant de conduire la ou les réaction(s) de condensation par estérification avec l'acide dicarboxylique ou un dérivé de celui-ci de formule (II). D'autres variantes comprennent la réaction de quaternarisation sur les composés intermédiaires obtenus pendant les réactions de condensation par estérification séquentielles ou alternées. Une ou plusieurs réaction(s) de quaternarisation totale(s) ou partielle(s) peuvent être conduites après l'une quelconque de ces étapes intermédiaires.

Les réactions de quaternarisation sont généralement conduites dans l'eau et/ou dans un ou des solvant(s) organique(s), tels que l'éthanol, l'isopropanol (IPA), l'éther monobutylique d'éthylène glycol, l'éther monobutylique de di(éthylène glycol) (BDG), le monoéthylène glycol (MEG), le diéthylène glycol (DEG), ou des mélanges de ceux-ci. Les solvants préférés sont choisis parmi l'isopropanol (IPA), l'éthanol, et des mélanges de ceux-ci.

La température de réaction de de quaternarisation est, de manière appropriée, dans la plage de 20 °C à 100°C, de préférence au moins 40 °C, plus préférablement au moins 50°C et, de manière préférée entre toutes, au moins 55 °C et, de préférence, au plus 90°C. La réaction de quaternarisation est typiquement conduite pendant une durée dans la plage de plusieurs dizaines de minutes à plusieurs dizaines d'heures, de préférence d'une heure à 100 heures, plus préférablement de 1 heure à 30 heures.

La réaction de quaternarisation peut être partielle ou totale. La surveillance de la réaction de quaternarisation est généralement effectuée au moyen de la diminution de l'alcalinité totale du milieu de réaction. Une « quaternarisation totale » est obtenue lorsque la valeur d'alcalinité totale est inférieure ou égale à 0,2 meqlg, de préférence inférieure ou égale à 0,1 meqlg, plus préférablement inférieure ou égale à 0,05 meqlg telle que mesurée par titrage avec de l'acide chlorhydrique.

Dans un mode de réalisation, des composés de formule (1) préférés sont ceux dans lesquels :

- $R^2$ est choisi dans le groupe constitué d'un radical hydrocarbyle divalent ayant de 1 à 10, de préférence de 2 à 6, et de manière préférée entre toutes 4 atomes de carbone, limites incluses,
- lorsque $R^{10}$ est $R^7$, $R^7$ est choisi parmi un groupe hydrocarbyle ayant 1 à 4 atomes de carbone, de préférence 1 à 2 atomes de carbone et de manière préférée entre toutes $R^7$ est méthyle,
- lorsque $R^{10}$ est $R^1$-(G)y-, y = 0 et $R^1$ est choisi parmi un groupe hydrocarbyle ayant 8 à 24 atomes de carbone, de préférence 12 à 24 atomes de carbone,
- QO représente un groupe éthoxy et
- p, $q_1$, $q_2$, $q_3$, $q_4$, t, $R^5$ sont tels que définis ci-dessus.

Selon un autre mode de réalisation, des composés de formule (1) préférés sont ceux dans lesquels tous les "t" sont égaux à 1, c'est-à-dire que tous les atomes d'azote sont quaternarisés (ce qui signifie une « quarternarisation totale »), tous les autres groupes et entiers variables étant tels que définis ci-dessus.

Selon un autre mode de réalisation, des composés de formule (1) préférés sont ceux dans lesquels tous les "t" sont égaux à 1, et $R^5$ est choisi parmi méthyle et éthyle, tous les autres groupes et entiers variables étant tels que définis ci-dessus.

Selon un autre mode de réalisation supplémentaire, des composés de formule (1) préférés sont ceux dans lesquels tous les "t" sont égaux à 1, $R^5$ est choisi parmi méthyle et éthyle, et X est choisi parmi des halogènes et des

sulfates (par exemple, des méthosulfates), tous les autres groupes et entiers variables étant tels que définis ci-dessus.

Selon un autre mode de réalisation, des composés de formule (1) préférés sont ceux dans lesquels $q_1$, $q_2$, $q_3$ and $q_4$, indépendamment les uns des autres, sont identiques ou différents, et sont choisis parmi 1, 2, 3, 4, 5 et 6, p est dans la plage de 1 à 10, limites incluses, et tous les autres groupes variables et entiers sont tels que définis ci-dessus.

Des composés de formule (1) particulièrement préférés selon la présente invention sont ceux obtenus par réaction(s) de condensation par estérification simultanée(s)/séquentielle(s)/alternée(s) de :

- au moins un composé de formule (I), dans lequel y = 0, et $R^1$ est choisi parmi un groupe hydrocarbyle ayant 8 à 24 atomes de carbone, de préférence 10 à 24, plus préférablement 12 à 24 atomes de carbone,
- au moins un composé de formule (II), et
- au moins un composé de formule (III), dans lequel $R^7$ est un groupe hydrocarbyle ayant 1 à 7, de préférence 1 à 6 atomes de carbone, plus préférablement 1 à 4 atomes de carbone,

ainsi que leurs produits de réaction de quaternarisation partielle ou totale.

Les composés de formule (1) préférés entre tous selon la présente invention sont ceux obtenus par réaction(s) de condensation par estérification simultanée(s)/séquentielle(s)/alternée(s) de :

- au moins un composé de formule (I), dans lequel y = 0, $R^1$ est choisi parmi un groupe hydrocarbyle ayant 8 à 24 atomes de carbone, de préférence 10 à 24, plus préférablement 12 à 24 atomes de carbone et AO est éthoxy,
- au moins un composé de formule (II), et
- au moins un composé de formule (III), dans lequel $R^7$ est un groupe hydrocarbyle ayant 1 à 7, de préférence 1 à 6 atomes de carbone, plus préférablement 1 à 4 atomes de carbone, et A"O est éthoxy,

ainsi que leurs produits de réaction de quaternarisation partielle ou totale.

Des composés de formule (1) particulièrement préférés entre tous selon la présente invention sont ceux obtenus par réaction(s) de condensation par estérification simultanée(s)/séquentielle(s)/alternée(s) de :

- au moins un composé de formule (I), dans lequel y = 0, $R^1$ est choisi parmi un groupe hydrocarbyle ayant 8 à 24 atomes de carbone, de préférence 10 à 24, plus préférablement 12 à 24 atomes de carbone, AO est éthoxy, et m et n, chacun indépendamment l'un de l'autre, identiques ou différents, représentent un entier compris entre 1 à 10, de préférence 1 à 6, plus préférablement 1 à 4, limites incluses,
- au moins un composé de formule (II), choisi parmi des diacides (D représente - OH) et leurs anhydrides correspondants, dans lequel $R^2$ est un radical hydrocarbyle divalent ayant de 1 à 14, plus préférablement de 1 à 10, encore plus préférablement de 1 à 8 atomes de carbone, limites incluses, et
- au moins un composé de formule (III), dans lequel $R^7$ est un groupe hydrocarbyle ayant 1 à 7, de préférence 1 à 6 atomes de carbone, plus préférablement 1 à 4 atomes de carbone, A"O est éthoxy, et u et u' représentent chacun 1,

ainsi que leurs produits de réaction de quaternarisation totale avec le chlorure de méthyle.

Des composés de formule (1) préférés typiques selon la présente invention sont ceux obtenus par réaction(s) de condensation par estérification simultanée(s)/séquentielle(s)/alternée(s) de :

- au moins un composé de formule (I) choisi parmi la n-octylamine, la n-décylamine, la n-dodécylamine, la (coco-alkyl)amine, la (huile de palme-alkyl)amine, la n-tétradécylamine, la n-hexadécylamine, la n-octadécylamine, l'oléylamine, la (suif-alkyl)amine, la (suif hydrogéné-alkyl)amine, la (colza-alkyl)amine, la (soja-alkyl)amine, l'érucylamine, alcoxylées avec 2 à 20, de préférence 2 à 10 EO (motifs d'oxyde d'éthylène), et/ou 2 à 20, de préférence 2 à 10 PO (motifs d'oxyde de propylène),
- au moins un composé de formule (II) choisi parmi l'acide malonique, l'acide succinique, l'acide glutarique, l'acide glutaconique, l'acide adipique, l'acide muconique, l'acide pimélique, l'acide phtalique et ses isomères, l'acide tétrahydrophtalique, l'acide malique, l'acide maléique, l'acide fumarique, l'acide subérique, l'acide mésaconique, l'acide sébacique, l'acide azélaïque, l'acide tartrique, l'acide itaconique, l'acide glutinique, l'acide citraconique, l'acide brassylique, l'acide dodécanedioïque, l'acide traumatique, l'acide thapsique, leurs chlorures d'acide correspondants, leurs esters méthyliques ou éthyliques correspondants, et leurs anhydrides cycliques correspondants, ainsi que des mélanges de ceux-ci, et
- au moins un composé de formule (III) choisi parmi la méthyldiéthanolamine, l'éthyldiéthanolamine, la propyldiéthanolamine, la butyldiéthanolamine, l'isobutyldiéthanolamine, la pentyldiéthanolamine, l'hexyldiéthanolamine, l'heptyldiéthanolamine, ainsi que leurs produits d'alcoxylation correspondants,

ainsi que leurs produits de réaction de quaternarisation partielle ou totale.

Des composés de formule (1) plus préférés selon la présente invention sont ceux obtenus par réaction(s) de condensation simultanée(s)/séquentielle(s)/alternée(s) par estérification de :

- au moins un composé de formule (I) choisi parmi la n-dodécylamine, la (coco-alkyl)amine, la (huile de palme-alkyl)amine, la n-tétradécylamine, la n-hexadécylamine, la n-octadécylamine, l'oléylamine, la (suif-alkyl)amine, la (suif hydrogéné-alkyl)amine, la (colza-alkyl)amine, la (soja-alkyl)amine, l'érucylamine, alcoxylées avec 2 à 20, de préférence 2 à 10 EO (motifs d'oxyde d'éthylène),
- au moins un composé de formule (II) choisi parmi l'acide malonique, l'acide succinique, l'acide glutarique, l'acide glutaconique, l'acide adipique, l'acide muconique, l'acide pimélique, l'acide phtalique et ses isomères, l'acide tétrahydrophtalique, l'acide malique, l'acide maléique, l'acide fumarique, l'acide subérique, l'acide mésaconique, l'acide sébacique, l'acide azélaïque, l'acide tartrique, l'acide itaconique, l'acide glutinique, l'acide citraconique, l'acide brassylique, l'acide dodécanedioïque, l'acide traumatique, l'acide thapsique, leurs chlorures d'acide correspondants, leurs esters méthyliques ou éthyliques correspondants, et leurs anhydrides cycliques correspondants, ainsi que des mélanges de ceux-ci, et
- au moins un composé de formule (III) choisi parmi la méthyldiéthanolamine, l'éthyldiéthanolamine, la propyldiéthanolamine, la butyldiéthanolamine, l'isobutyldiéthanolamine, la pentyldiéthanolamine, l'hexyldiéthanolamine, l'heptyldiéthanolamine, ainsi que leurs produits d'éthoxylation correspondants,

ainsi que leurs produits de réaction de quaternarisation partielle ou totale.

Les composés de formule (1) préférés entre tous selon la présente invention sont ceux obtenus par réaction(s) de condensation par estérification simultanée(s)/séquentielle(s)/alternée(s) de :

- au moins un composé de formule (I) choisi parmi la n-dodécylamine, la (coco-alkyl)amine, la (huile de palme-alkyl)amine, la n-tétradécylamine, la n-hexadécylamine, la n-octadécylamine, l'oléylamine, la (suif-alkyl)amine, la (suif hydrogéné-alkyl)amine, la (colza-alkyl)amine, la (soja-alkyl)amine, l'érucylamine, alcoxylées avec 2 à 20, de préférence 2 à 10 EO (motifs d'oxyde d'éthylène),
- au moins un composé de formule (II) choisi parmi l'acide malonique, l'acide succinique, l'acide glutarique, l'acide adipique, l'acide pimélique, l'acide maléique, l'acide fumarique, l'acide subérique, l'acide sébacique, l'acide azélaïque, l'acide brassylique, l'acide dodécanedioïque, leurs anhydrides cycliques correspondants, et
- au moins un composé de formule (III) choisi parmi la méthyldiéthanolamine, l'éthyldiéthanolamine, la propyldiéthanolamine, la butyldiéthanolamine, l'isobutyldiéthanolamine, la pentyldiéthanolamine, l'hexyldiéthanolamine, l'heptyldiéthanolam ine,

ainsi que leurs produits de réaction de quaternarisation totale avec le chlorure de méthyle.

Les composés de formule (1) selon l'invention présentent de nombreux avantages, et parmi ces avantages, il peut être mentionné leur processus de préparation qui est relativement facile à mettre en oeuvre, même à une échelle industrielle. La plupart des composés de formule (1) peuvent être préparés aisément à partir de matériaux de départ peu coûteux et aisément disponibles.

Le composé pouvant être obtenu par la condensation par estérification de l'amine grasse alcoxylée de formule (I) (comprenant son produit de quaternarisation partielle ou totale), avec l'acide dicarboxylique, ou un dérivé de celui-ci, de formule (II), et avec un dérivé de (alkyl)alcanolamine de formule (III) (comprenant son produit de quaternarisation partielle ou totale), et plus particulièrement le composé de formule (1), tel que défini ci-dessus et selon la présente invention possèdent de nombreuses propriétés intéressantes. En tant que tel, il peut être utilisé, à titre d'illustration uniquement, et sans aucune limitation, en tant qu'agent tensioactif, biocide, agent anticorrosion, agent mouillant, et similaire.

En tant que tel, le composé pouvant être obtenu par la condensation par estérification de l'amine grasse alcoxylée de formule (I) (comprenant son produit de quaternarisation partielle ou totale), avec l'acide dicarboxylique, ou un dérivé de celui-ci, de formule (II), et avec un dérivé de (alkyl)alcanolamine de formule (III) (comprenant son produit de quaternarisation partielle ou totale), et plus particulièrement le composé de formule (1) tel que défini ci-dessus peut être utilisé dans différentes applications parmi lesquelles il peut être mentionné des utilisations en tant que collecteur pour l'enrichissement (flottation) de minerai, en tant qu'inhibiteur de corrosion, par exemple dans le domaine de la production de pétrole et de gaz, en tant que viscosificateur, émulsifiant ou stabilisant utile pour l'industrie du pétrole et du gaz (tel qu'un agent désémulsifiant, tensioactif pour améliorer la récupération de pétrole, tensioactif pour des fluides de fracturation, tensioactif pour des fluides d'acidification, tensioactif pour améliorer l'injectivité d'eau produite, tensioactif pour des boues de forage, et similaire), en tant que modificateur d'argile, en tant que promoteur d'adhérence, en tant qu'additif antiagglomérant, par exemple dans le domaine de la production de pétrole et de gaz, en tant qu'additif dans des produits de soin capillaire, en tant qu'assouplissant de tissu, en tant qu'agent antistatique dans des polymères, en tant qu'additif d'émulsion de bitume, en tant qu'agent cationique de détergence, par exemple dans le domaine de la

détergence industrielle, le lavage de voiture, en tant qu'additif pour des engrais, en tant qu'antiagglomérant pour des hydrates, en tant qu'additif de lubrification ou de promoteur d'adhérence, et similaire.

L'invention apparaîtra plus clairement au moyen des exemples suivants, qui sont présentées à titre d'illustration uniquement, sans aucune intention de limiter la portée de la protection recherchée définie par les revendications annexées. Dans l'ensemble de la description, des exemples et des revendications, toutes les plages de valeurs doivent être entendues comme étant « limites incluses » (c'est-à-dire que les limites sont incluses dans lesdites plages), sauf indication contraire spécifique.

**Procédé de mesure d'indice d'acide** :

Dans tous les exemples suivants, l'indice d'acide est mesuré par titrage potentiométrique en utilisant une solution d'hydroxyde de potassium en tant que réactif et l'alcool isopropylique en tant que solvant.

Dans un bécher de 250 ml, environ 10 g d'échantillon à analyser sont précisément pesés (Sw, précision au mg) et 70 ml d'alcool isopropylique sont ajoutés. Le mélange est agité et doucement chauffé, si nécessaire, pour obtenir un échantillon homogène. L'électrode de verre de référence combinée du titreur est introduite dans la solution, qui est ensuite agitée avec un agitateur magnétique. Le titrage acido-basique de l'échantillon est effectué en utilisant une solution aqueuse d'hydroxyde de potassium (KOH) 0,1 N et l'évolution du pH est enregistrée sur le titreur. Le point équivalent est déterminé graphiquement en utilisant des procédés connus de l'homme du métier, et le volume (VKOH, en ml) de solution d'hydroxyde de potassium utilisé pour atteindre ce point est déterminé. L'indice d'acide (AV) est ensuite obtenu selon le calcul suivant :

$$AV = \frac{[\text{Normalité de la solution de KOH (mol/L)}] \times 56{,}1 \times V_{KOH}}{Sw}$$

**Procédé de mesure d'alcalinité totale** :

Dans tous les exemples suivants, la valeur d'alcalinité totale est mesurée par titrage potentiométrique en utilisant une solution d'acide chlorhydrique en tant que réactif et l'alcool isopropylique en tant que solvant.

Dans un bécher en polypropylène de 100 ml, environ 3 g d'échantillon à analyser sont précisément pesés (précision au mg, Sw au g) et 60 ml d'alcool isopropylique sont ajoutés. Le mélange est agité et doucement chauffé, si nécessaire, pour obtenir un échantillon homogène. Une fois que la température de la solution est revenue à température ambiante, l'électrode de verre de référence combinée du titreur est introduite dans la solution, qui est ensuite agitée avec un agitateur magnétique. Le titrage de l'échantillon est effectué en utilisant une solution d'acide chlorhydrique (HCl) 0,2 N de normalité précisément connue (N, en $m_{eq}.ml^{-1}$) l'évolution du pH est enregistrée sur le titreur. Le point équivalent est déterminé en utilisant des procédés connus de l'homme du métier, et le volume (VHCl, en ml) de solution d'acide chlorhydrique utilisé pour atteindre ce point est déterminé. La valeur d'alcalinité totale (Alk) est ensuite obtenue selon le calcul suivant :

$$Alk\ (meq/g) = \frac{VHCL * n}{Sw}$$

**EXEMPLE 1 : synthèse d'un produit A (selon l'invention)**

Dans un ballon à fond rond de 4 l sont introduits 1196,7 g (2,5 moles) de suif-amine éthoxylée (5OE) fournie par Arkema sous le nom commercial Noramox® S5, 715,2 g (3,45 moles) de méthyldiéthanolamine (> 99 %) fournie par Taminco et 0,5 g d'une solution aqueuse d'acide hypophosphoreux à 50 % en poids.

Le mélange est chauffé à 80 °C avec barbotage d'azote. Le barbotage est arrêté et 756,8 g (5,18 moles) d'acide adipique sont ensuite introduits sous agitation.

Après 15 minutes, la température du mélange est augmentée à 120 °C en une durée de 1 heure et la pression dans la cuve est progressivement diminuée jusqu'à ce qu'une pression de 6,66 kPa (50 mm Hg) soit atteinte. La température est augmentée à 190 °C et la température et la pression sont maintenues jusqu'à ce que pratiquement tout l'acide soit consommé (indice d'acide < 5).

Le système est ensuite refroidi pour récupérer 2482,3 g de produit de réaction liquide brut orange/brun contenant l'esteramine souhaitée, les amines n'ayant pas réagi et le diacide n'ayant pas réagi.

**EXEMPLE 2** : **synthèse d'un produit B (selon l'invention)**

Dans un ballon à fond rond de 4 l sont introduits 1420,1 g (2,97 moles) de suif-amine éthoxylée (5OE) fournie par Arkema sous le nom commercial Noramox® S5, 353,2 g (2,97 moles) de méthyldiéthanolamine (> 99 %) fournie par Taminco et 0,5 g d'une solution aqueuse d'acide hypophosphoreux à 50 % en poids.

Le mélange est chauffé à 80 °C avec barbotage d'azote. Le barbotage est arrêté et 650 g (4,45 moles) d'acide adipique sont ensuite introduits sous agitation.

Après 15 minutes, la température du mélange est augmentée à 160 °C et maintenue pendant 4 heures. La température est ensuite augmentée à 190 °C et la température et la pression sont maintenues jusqu'à ce que pratiquement tout l'acide soit consommé (indice d'acide < 5). La pression dans la cuve est ensuite progressivement diminuée jusqu'à ce qu'une pression de 6,66 kPa (50 mm Hg) soit atteinte et la température et la pression sont maintenues pendant 2 heures supplémentaires.

Finalement, le système est refroidi et la pression est ramenée à la pression atmosphérique afin de récupérer 2260 g de produit de réaction liquide brut orange/brun contenant l'esteramine souhaitée, les amines n'ayant pas réagi et le diacide n'ayant pas réagi.

**EXEMPLE 3 : synthèse d'un produit C (selon l'invention)**

Dans un réacteur en verre de 6 l, sont introduits 2022 g du produit d'esteramine A obtenu dans l'exemple 1 avec 453 g d'alcool isopropylique. Du chlorure de méthyle est ajouté jusqu'à ce que la pression dans la cuve atteigne 290 kPa. La température est maintenue à 80°C - 85 °C jusqu'à ce qu'une réaction totale se soit produite.

Une réaction totale est obtenue lorsque la valeur d'alcalinité totale est inférieure ou égale à 0,2 $m_{eq}.g^{-1}$. On laisse ensuite le réacteur refroidir à 65 °C et la pression est ramenée à la pression atmosphérique. On fait barboter de l'azote pendant 2 heures dans le mélange avant de récupérer 2095,9 g du produit de réaction brut brun contenant encore 6,7 % en poids d'alcool isopropylique.

**EXEMPLE 4 : synthèse d'un produit D (selon l'invention)**

Dans un réacteur en verre de 6 l, sont introduits 1803,7 g du produit d'esteramine B obtenu dans l'exemple 2 avec 788,3 g d'alcool isopropylique. Du chlorure de méthyle est ajouté jusqu'à ce que la pression dans la cuve atteigne 290 kPa. La température est maintenue à 80 °C - 85 °C jusqu'à ce qu'une réaction totale se soit produite.

Une réaction totale est obtenue lorsque la valeur d'alcalinité totale est inférieure ou égale à 0,2 $m_{eq}.g^{-1}$. On laisse ensuite le réacteur refroidir à 65 °C et la pression est ramenée à la pression atmosphérique. On fait barboter de l'azote pendant 2 heures dans le mélange avant de récupérer 2206,6 g du produit de réaction brut brun contenant encore 17,3 % en poids d'alcool isopropylique.

**EXEMPLE 5** : **synthèse d'un produit E (selon l'invention)**

Dans un autoclave sec de 4 l, 595 g (5 M) de MDEA (méthyldiéthanolamine) et 6 g de KOH (solution aqueuse à 50 % en poids) sont ajoutés. Le réacteur est ensuite fermé et rempli avec une atmosphère d'azote et le joint est protégé contre les fuites. Le MDEA et le catalyseur sont déshydratés à moins de 1000 ppm d'eau. La pression est ensuite augmentée à 75 kPa et 25 °C avec de l'azote. La température dans le réacteur est ensuite augmentée à 90 °C sous agitation. Ensuite, la température est à nouveau augmentée à 120 °C et 40 g à 50 g d'oxyde d'éthylène sont ajoutés. De l'oxyde d'éthylène additionnel, pour un total de 1100 g (25 M) au total, est ajouté pendant 3 heures à 140 °C - 150 °C. Après l'ajout de l'oxyde d'éthylène, le mélange de réaction est maintenu à cette température pendant 30 min (« cuisson »), puis la phase liquide est soumise à une distillation sous azote. À la fin de la réaction, le réacteur est refroidi à 60 °C et 1655 g de MDEA 5 EO sont obtenus.

Dans un ballon à fond rond de 4 l sont introduits 1420,3 g (2,97 moles) de suif-amine éthoxylée (5OE) fournie par Arkema sous le nom commercial Noramox® S5, 745,5 g (2,97 moles) de MDEA 5OE (synthétisé comme décrit ci-dessus) et 0,5 g d'une solution aqueuse d'acide hypophosphoreux à 50 % en poids.

Le mélange est chauffé à 80 °C avec barbotage d'azote. Le barbotage est arrêté et 650 g (4,45 moles) d'acide adipique sont ensuite introduits sous agitation.

Après 15 minutes, la température du mélange est augmentée à 160 °C et maintenue pendant 4 heures. La température est ensuite augmentée à 190 °C et la température et la pression sont maintenues jusqu'à ce que pratiquement tout l'acide soit consommé (indice d'acide < 5). La pression dans la cuve est ensuite progressivement diminuée jusqu'à ce qu'une pression de 6,66 kPa (50 mm Hg) soit atteinte et la température et la pression sont maintenues pendant 2 heures supplémentaires.

Finalement, le système est refroidi et la pression est ramenée à la pression atmosphérique afin de récupérer

2654,5 g de produit de réaction liquide brut orange/brun contenant l'esteramine souhaitée, les amines n'ayant pas réagi et le diacide n'ayant pas réagi.

**EXEMPLE 6** : **synthèse d'un produit F (selon l'invention)**

Dans un réacteur en verre de 6 l, sont introduits 2050 g du produit d'esteramine E obtenu dans l'exemple 5 avec 615 g d'alcool isopropylique. Du chlorure de méthyle est ajouté jusqu'à ce que la pression dans la cuve atteigne 290 kPa. La température est maintenue à 80°C - 85 °C jusqu'à ce qu'une réaction totale se soit produite.

Une réaction totale est obtenue lorsque la valeur d'alcalinité totale est inférieure ou égale à 0,2 $m_{eq}.g^{-1}$. On laisse ensuite le réacteur refroidir à 65 °C et la pression est ramenée à la pression atmosphérique. On fait barboter de l'azote pendant 2 heures dans le mélange avant de récupérer 2496,9 g du produit de réaction brut brun contenant encore 12,4 % en poids d'alcool isopropylique.

**EXEMPLE 7** : **synthèse d'un produit G (selon l'invention)**

Dans un autoclave sec de 4 l, 510 g (5 M) de DMAPA (diméthylaminopropylamine) et 5 g (1 % en poids) d'eau sont ajoutés. Le réacteur est ensuite fermé et rempli avec une atmosphère d'azote et le joint est protégé contre les fuites. La pression est ensuite augmentée à 100 kPa et 30°C avec de l'azote. La température dans le réacteur est ensuite augmentée à 120 °C sous agitation. 40 g d'oxyde d'éthylène sont ajoutés. La température est augmentée linéairement jusqu'à ce que la réaction commence. De l'oxyde d'éthylène additionnel, pour un total de 1100 g (25 M) au total, est ajouté pendant 4 heures à 150 °C - 160 °C. Après l'ajout de l'oxyde d'éthylène, le mélange de réaction est maintenu à cette température pendant 30 min (« cuisson »), puis la phase liquide est soumise à une distillation sous azote. À la fin de la réaction, le réacteur est refroidi à 60 °C et 1570 g de DMAPA 5 EO sont obtenus.

Dans un ballon à fond rond de 4 l sont introduits 1196,1 g (2,5 moles) de suif-amine éthoxylée (5OE) fournie par Arkema sous le nom commercial Noramox® S5, 805,4 g (2,5 moles) de DMAPA 5OE (synthétisé comme décrit ci-dessus) et 0,5 g d'une solution aqueuse d'acide hypophosphoreux à 50 % en poids.

Le mélange est chauffé à 80 °C avec barbotage d'azote. Le barbotage est arrêté et 547,9 g (3,75 moles) d'acide adipique sont ensuite introduits sous agitation.

Après 15 minutes, la température du mélange est augmentée à 160 °C et maintenue pendant 4 heures. La température est ensuite augmentée à 190 °C et la température et la pression sont maintenues jusqu'à ce que pratiquement tout l'acide soit consommé (indice d'acide < 5). La pression dans la cuve est ensuite progressivement diminuée jusqu'à ce qu'une pression de 6,66 kPa (50 mm Hg) soit atteinte et la température et la pression sont maintenues pendant 2 heures supplémentaires.

Finalement, le système est refroidi et la pression est ramenée à la pression atmosphérique afin de récupérer 2413,9 g de produit de réaction liquide brut orange/brun contenant l'esteramine souhaitée, les amines n'ayant pas réagi et le diacide n'ayant pas réagi.

**EXEMPLE 8** : **synthèse d'un produit H (selon l'invention)**

Dans un réacteur en verre de 6 l, sont introduits 2040 g du produit d'esteramine E obtenu dans l'exemple 5 avec 600 g d'alcool isopropylique. Du chlorure de méthyle est ajouté jusqu'à ce que la pression dans la cuve atteigne 290 kPa. La température est maintenue à 80°C - 85 °C jusqu'à ce qu'une réaction totale se soit produite.

Une réaction totale est obtenue lorsque la valeur d'alcalinité totale est inférieure ou égale à 0,2 $m_{eq}.g^{-1}$. On laisse ensuite le réacteur refroidir à 65 °C et la pression est ramenée à la pression atmosphérique. On fait barboter de l'azote pendant 2 heures dans le mélange avant de récupérer 2396,7 g du produit de réaction brut brun contenant encore 12,9 % en poids d'alcool isopropylique.

**EXEMPLE 9 : synthèse des produits I à R (selon l'invention)**

Selon le même procédé que dans l'exemple 1, les produits suivants ont été préparés à partir des composés indiqués dans le tableau 1 ci-dessous :

**Tableau 1 :** composés et quantités utilisés pour synthétiser les produits I à R selon l'invention

| Amine grasse alcoxylée de formule (I) | Poids de (I) (en g) | Acide dicarboxylique, ou dérivé, de formule (II) | Poids de (II) (en g) | Dérivé de (alkyl)-alcanolamine de formule (III) | Poids de (III) (en g) | Produit | Poids de produit (en g) |
|---|---|---|---|---|---|---|---|
| NoxS5 | 717,7 | Ac. Ad. | 657,5 | MDEA | 932,9 | I | 2145,6 |
| NoxS5 | 956,9 | Ac. Ad. | 730,5 | MDEA | 829,2 | J | 2336,1 |
| NoxS5 | 1435,4 | Anh. succ. | 450,5 | MDEA | 621,9 | K | 2345,4 |
| NoxS5 | 1435,4 | Anh. Malé. | 441,5 | MDEA | 621,9 | L | 2336,2 |
| NoxS5 | 1435,4 | Ac.Séb. | 909,9 | MDEA | 621,9 | M | 2804,5 |
| NoxS2 | 1039,4 | Ac. Ad. | 657,5 | MDEA | 621,9 | N | 2156,3 |
| NoxS11 | 1446,4 | Ac. Ad. | 438,3 | MDEA | 414,6 | O | 2190,8 |
| NoxC5 | 1320,9 | Ac. Ad. | 657,5 | MDEA | 621,9 | P | 2437,7 |
| NoxS5 | 1435,4 | Ac. Ad. | 657,5 | TEA | 447,6 | Q | 2378,1 |
| NoxS11 | 1446,4 | Ac. Ad. | 438,3 | MDEA5OE | 502,0 | R | 2278,2 |

NoxS5 est une suif-amine éthoxylée (5OE) fournie par Arkema sous le nom commercial Noramox® S5
NoxS2 est une suif-amine éthoxylée (2OE) fournie par Arkema sous le nom commercial Noramox® S2
NoxS11 est une suif-amine éthoxylée (110E) fournie par Arkema sous le nom commercial Noramox® S11
NoxC5 est une coco-amine éthoxylée (5OE) fournie par Arkema sous le nom commercial Noramox® C5
MDEA est la méthyldiéthanolamine (> 99 %) fournie par Taminco
TEA est la triéthanolamine (> 99 %) fournie par Taminco
MDEA est la méthyldiéthanolamine (> 99 %) fournie par Taminco
MDEA 5 OE est tel que décrit dans l'exemple 5
Ac. Ad. désigne l'acide adipique, Anh. Succ. désigne l'anhydride succinique et Anh. Malé. désigne l'anhydride maléique.

**EXEMPLE 10 : synthèse des produits 5 à AB (selon l'invention)**

Selon le même procédé que dans l'exemple 3, les dérivés d'ammonium quaternaire de chlorométhyle des produits I à R ont été préparés à partir des composés indiqués dans le tableau 2 ci-dessous :

**Tableau 2 :** composés et quantités utilisés pour synthétiser les produits S à AB selon l'invention

| Produit réactif | Poids de produit (en g) | Poids d'isopropanol (en g) | Produit obtenu | Poids de produit obtenu (en g) | Teneur en isopropanol du produit (% en poids) |
|---|---|---|---|---|---|
| I | 1800,2 | 540,1 | S | 2187,7 | 14,8 % |
| J | 1900,5 | 570,2 | T | 2255,1 | 13,9 % |
| K | 1900,2 | 570,1 | U | 2286,4 | 14,2 % |
| L | 1900,1 | 570,0 | V | 2258,0 | 13,1 % |
| M | 2200,3 | 660,1 | W | 2556,7 | 11,6 % |
| N | 1800,4 | 540,1 | X | 2176,8 | 14,4 % |
| 0 | 1800,1 | 540,0 | Y | 2111,6 | 12,8 % |
| P | 2000,1 | 600,0 | Z | 2350,2 | 12,3 % |
| Q | 2000,3 | 600,1 | AA | 2322,0 | 11,1 % |
| R | 1800,4 | 540,1 | AB | 2142,7 | 14,1 % |

## Revendications

**1.** Composé pouvant être obtenu par la condensation par estérification de :

A/ une amine grasse alcoxylée de formule (I), ou du produit de quaternarisation partielle ou totale de ladite amine grasse alcoxylée de formule (I) :

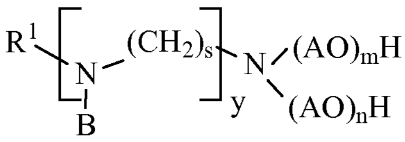

(I)

dans laquelle :

- $R^1$ est choisi parmi un groupe hydrocarbyle ayant 8 à 24 atomes de carbone, de préférence 10 à 24, plus préférablement 12 à 24 atomes de carbone, et un groupe de formule $R^4$-O-(A'O)$_w$-T-, dans laquelle $R^4$ est un groupe hydrocarbyle ayant 8 à 24 atomes de carbone, de préférence 12 à 24 atomes de carbone, w représente un entier dans la plage de 0 à 20, de préférence de 0 à 10, plus préférablement de 0 à 6, et encore plus préférablement de 0 à 4, A'O est un groupe alkylénoxy contenant 2 à 4 atomes de carbone, de préférence 2 ou 3 atomes de carbone, plus préférablement 2 atomes de carbone ; T est alkylène avec 1 à 6 atomes de carbone, de préférence 1 à 4 atomes de carbone, de manière préférée entre toutes 2 ou 3 atomes de carbone,
- AO est un groupe alkylénoxy contenant 2 à 4 atomes de carbone, de préférence 2 ou 3 atomes de carbone, plus préférablement 2 atomes de carbone,
- B est choisi parmi un groupe alkyle en $C_1$-$C_4$, aryle ou arylalkyle (par exemple phényle, phénylalkyle, tel que benzyle),
- m représente un entier compris entre 1 et 20, de préférence entre 1 et 10, plus préférablement entre 1 et 6, et encore plus préférablement entre 1 et 4, limites incluses,
- n représente un entier compris entre 1 et 20, de préférence entre 1 et 10, plus préférablement entre 1 et 6, et encore plus préférablement entre 1 et 4, limites incluses,
- s est 1, 2 ou 3, de préférence 2 ou 3, et
- y est un entier de 0 à 5, de préférence de 0 à 3, plus préférablement y est 0 ou 1, encore plus préférablement y est 0,

B/ avec un acide dicarboxylique, ou un dérivé de celui-ci, de formule (II) :

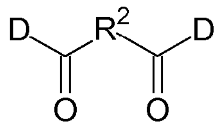

(II),

dans laquelle

- D est choisi parmi -F, -Cl, Br et -$OR^3$, où $R^3$ est hydrogène ou un groupe alkyle en $C_1$-$C_4$,
- $R^2$ est choisi dans le groupe constitué de :
  - ◦une liaison directe,
  - ◦ une chaîne hydrocarbonée linéaire ou ramifiée, saturée ou insaturée en $C_1$-$C_{20}$ facultativement substituée par un ou plusieurs groupe(s) -OH, de préférence un radical alkylène de formule $-(CH_2)_z-$, dans laquelle z est un entier de 1 à 20, de préférence de 1 à 10, de préférence de 2 à 6, et de manière préférée entre toutes de 4, un radical alkylène substitué, ledit radical alkylène étant substitué par 1 ou 2 groupes -OH, un radical alcénylène ayant de 1 à 20, de préférence de 1 à 10 atomes de carbone, un radical alcénylène substitué, ledit radical alcénylène étant substitué par 1 ou 2 groupes méthyle et/ou méthylène,
  - ◦ un groupe cycloalkylène,

◦ cycloalcénylène et
◦ arylène

C/ avec un dérivé de (alkyl)alcanolamine de formule (III) ou du produit de quaternarisation partielle ou totale dudit dérivé de (alkyl)alcanolamine de formule (III) :

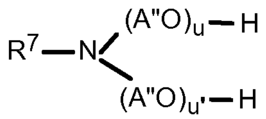

(III)

dans laquelle :

- A"O représente un groupe alkylénoxy contenant de 2 à 4 atomes de carbone, de préférence 2 ou 3 atomes de carbone, plus préférablement 2 atomes de carbone,
- u représente un entier compris entre 1 et 20, de préférence entre 1 et 10, plus préférablement entre 1 et 6, et encore plus préférablement entre 1 et 4, limites incluses,
- u' représente un entier compris entre 1 et 20, de préférence entre 1 et 10, plus préférablement entre 1 et 6, et encore plus préférablement entre 1 et 4, limites incluses,
- $R^7$ est choisi parmi un groupe hydrocarbyle ayant 1 à 7, de préférence 1 à 6 atomes de carbone, plus préférablement 1 à 4 atomes de carbone, un groupe aryle ou arylalkyle (par exemple, un groupe phényle ou naphtyle), un groupe de formule $H\text{-}(OA")_v\text{-}$ (dans laquelle v représente un entier compris entre 1 et 20, de préférence entre 1 et 10, plus préférablement entre 1 et 6, et encore plus préférablement entre 1 et 4, limites incluses), $HO(CH_2)_q\text{-}$ et un groupe de formule (IV) :

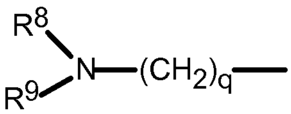

(IV),

dans laquelle $R^8$ et $R^9$, identiques ou différents, sont choisis parmi un groupe hydrocarbyle ayant 1 à 6 atomes de carbone, de préférence 1 à 4 atomes de carbone et q est un entier de 1 à 10, de préférence de 2 à 6, limites incluses, et de manière préférée entre toutes q est 2 ou 3,
ou $R^8$ et $R^9$, conjointement avec l'atome d'azote auquel ils sont liés forment un cycle de 5, 6 ou 7 atomes, comportant facultativement un ou plusieurs hétéroatome(s) choisi(s) parmi l'oxygène, l'azote ou le soufre.

**2.** Composé selon la revendication 1, dans lequel le radical $R^1$ de l'amine grasse de formule (I) comporte 8, ou plus de 8, atomes de carbone, typiquement de 8 à 24 atomes de carbone, de préférence de 10 à 24, plus préférablement de 12 à 24 atomes de carbone, limites incluses, et le radical $R^7$ du dérivé de (alkyl)alcanolamine de formule (III) comporte 6 atomes de carbone ou moins, typiquement de 1 à 6 atomes de carbone, plus préférablement 1 à 4 atomes de carbone, limites incluses.

**3.** Composé selon la revendication 1 ou la revendication 2, dans lequel le radical $R^1$ et le radical $R^7$ de l'amine grasse de formule (I) et du dérivé de (alkyl)alcanolamine de formule (III) respectivement, sont tels que la différence de nombre d'atomes de carbone qu'ils comportent est supérieure à 2, typiquement de 2 à 23, de préférence 5 à 23, plus préférablement 10 à 23, limites incluses.

**4.** Composé selon l'une quelconque des revendications 1 à 3, dans lequel une partie ou la totalité des atomes d'azote réagissent en outre avec un réactif de formule $R^5X$, dans laquelle $R^5$ est choisi parmi un groupe hydrocarbyle en $C_1\text{-}C_6$, de préférence un groupe alkyle en $C_1\text{-}C_4$, phényle et phénylalkyle, tel que benzyle, et X est choisi parmi des halogènes, des sulfates, des carbonates, et similaire.

**5.** Composé selon l'une quelconque des revendications 1 à 4, dans lequel l'amine grasse alcoxylée de formule (I) est de formule (IA) :

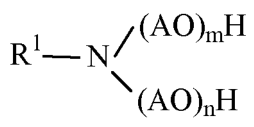

(IA)

qui est l'amine grasse alcoxylée de formule (I) dans laquelle y représente 0, et $R^1$, AO, m et n sont tels que définis dans la revendication 1,
ainsi que ses dérivés partiellement ou totalement quaternarisés correspondants.

**6.** Composé selon l'une quelconque des revendications 1 à 5, dans lequel le dérivé de (alkyl)alcanolamine de formule (III) est de formule (IIIA) :

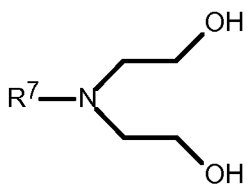

(IIIA)

qui est l'(alkyl)alcanolamine de formule (III) dans laquelle u et u' représentent chacun 1, A"O est éthylénoxy et $R^7$ est tel que défini dans la revendication 1, et est de préférence un groupe hydrocarbyle ayant 1 à 4 atomes de carbone,
ainsi que ses dérivés partiellement ou totalement quaternarisés correspondants.

**7.** Composé selon l'une quelconque des revendications 1 à 6, dans lequel les dérivés de (alkyl)alcanolamines de formule (III) comprennent, mais ne sont pas limités à, la triéthanolamine, la méthyldiéthanolamine, l'éthyldiéthanolamine, la propyldiéthanolamine, la butyldiéthanolamine, l'isobutyldiéthanolamine, la pentyldiéthanolamine, la phényldiéthanolamine, l'hexyldiéthanolamine, l'heptyldiéthanolamine, ainsi que leurs produits d'alcoxylation correspondants.

**8.** Composé selon l'une quelconque des revendications 1 à 7, dans lequel le dérivé d'acide dicarboxylique de formule générale (II) est choisi parmi un acide dicarboxylique, un halogénure d'acide dicarboxylique, un diester d'un acide dicarboxylique, ou un anhydride cyclique d'un acide dicarboxylique.

**9.** Composé selon l'une quelconque des revendications 1 à 8, dans lequel des exemples illustratifs de dérivés d'acide dicarboxylique de formule générale (II) comprennent l'acide oxalique, l'acide malonique, l'acide succinique, l'acide glutarique, l'acide glutaconique, l'acide adipique, l'acide muconique, l'acide pimélique, l'acide phtalique et ses isomères, l'acide tétrahydrophtalique, l'acide malique, l'acide maléique, l'acide fumarique, l'acide subérique, l'acide mésaconique, l'acide sébacique, l'acide azélaïque, l'acide tartrique, l'acide itaconique, l'acide glutinique, l'acide citraconique, l'acide brassylique, l'acide dodécanedioïque, l'acide traumatique, l'acide thapsique, leurs chlorures d'acide correspondants, leurs esters méthyliques ou éthyliques correspondants, et leurs anhydrides cycliques correspondants, ainsi que des mélanges de ceux-ci.

**10.** Composé selon l'une quelconque des revendications 1 à 9, dans lequel le rapport molaire entre les réactifs [(I) + (III)] et (II) est de 2:1 à 1:2, de préférence 1,5:1 à 1:1,5, et de manière préférée entre toutes 1,4:1 à 1:1,4.

**11.** Composé selon l'une quelconque des revendications 1 à 9, dans lequel le rapport molaire entre les réactifs [(I) + (III)] et (II) est de 2:1 à 1:1, de préférence 2:1 à 1,2:1, et de manière préférée entre toutes 2:1 à 1,3:1.

**12.** Composé selon l'une quelconque des revendications 1 à 11, dans lequel le rapport molaire entre (I) et (III) est de 15:1 à 1:15, de préférence 10:1 à 1:10, plus préférablement 4:1 à 1:4, et de manière préférée entre toutes 2:1 à 1:2.

**13.** Composé de formule générale (1) :

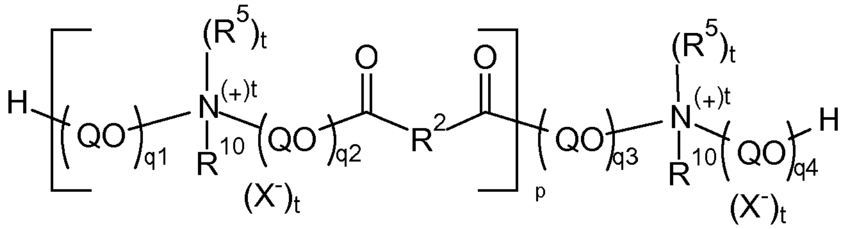

(1)

dans laquelle :

- $R^2$ est choisi dans le groupe constitué de :

◦une liaison directe,
◦une chaîne hydrocarbonée linéaire ou ramifiée, saturée ou insaturée en $C_1$-$C_{20}$ facultativement substituée par un ou plusieurs groupe(s) -OH, de préférence un radical alkylène de formule $-(CH_2)_z-$, dans laquelle z est un entier de 1 à 20, de préférence de 1 à 10, de préférence de 2 à 6, et de manière préférée entre toutes de 4, un radical alkylène substitué, ledit radical alkylène étant substitué par 1 ou 2 groupes -OH, un radical alcénylène ayant de 1 à 20, de préférence de 1 à 10 atomes de carbone, un radical alcénylène substitué, ledit radical alcénylène étant substitué par 1 ou 2 groupes méthyle et/ou méthylène,
◦un groupe cycloalkylène,
◦cycloalcénylène et
◦arylène

- $R^5$ est choisi parmi un groupe hydrocarbyle en $C_1$-$C_6$, de préférence un groupe alkyle en $C_1$-$C_4$, phényle et phénylalkyle, tel que benzyle.
- X est choisi parmi des halogènes, des sulfates, des carbonates, et similaire,
- test 0 ou 1
- p est un entier dans la plage de 1 à 15, de préférence de 1 à 10, plus préférablement de 1 à 5, limites incluses,
- QO représente un groupe alkylénoxy contenant de 2 à 4 atomes de carbone, de préférence 2 ou 3 atomes de carbone, plus préférablement 2 atomes de carbone, sachant que tous les Q présents dans le composé de formule (1) peuvent être identiques ou différents,
- $q_1$, $q_2$, $q_3$, $q_4$, identiques ou différents les uns des autres, représentent chacun un entier compris entre 1 et 20, de préférence entre 1 et 10, plus préférablement entre 1 et 6, et encore plus préférablement entre 1 et 4, limites incluses,
- chaque groupe $R^{10}$, indépendamment des autres, représente $R^7$ ou un groupe $R^1$-$(G)_y$-,
- $R^7$ est choisi parmi un groupe hydrocarbyle ayant 1 à 7, de préférence 1 à 6 atomes de carbone, plus préférablement 1 à 4 atomes de carbone, un groupe aryle ou arylalkyle (par exemple, un groupe phényle ou naphtyle), un groupe de formule H-$(OA")_v$- (dans laquelle v représente un entier compris entre 1 et 20, de préférence entre 1 et 10, plus préférablement entre 1 et 6, et encore plus préférablement entre 1 et 4, limites incluses), $HO(CH_2)_q$- et un groupe de formule (IV) :

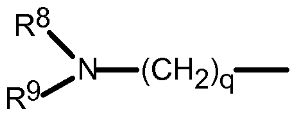

(IV),

dans laquelle $R^8$ et $R^9$, identiques ou différents, sont choisis parmi un groupe hydrocarbyle ayant 1 à 6 atomes de carbone, de préférence 1 à 4 atomes de carbone et q est un entier de 1 à 10, de préférence de 2 à 6, limites incluses, et de manière préférée entre toutes q est 2 ou 3,
- ou $R^8$ et $R^9$, conjointement avec l'atome d'azote auquel ils sont liés forment un cycle de 5, 6 ou 7 atomes, comportant facultativement un ou plusieurs hétéroatome(s) choisi(s) parmi l'oxygène, l'azote ou le soufre.
- $R^1$ est choisi parmi un groupe hydrocarbyle ayant 8 à 24 atomes de carbone, de préférence 10 à 24, plus préférablement 12 à 24 atomes de carbone, et un groupe de formule $R^4$-O-$(A'O)_w$-T-, dans laquelle $R^4$ est un groupe hydrocarbyle ayant 8 à 24 atomes de carbone, de préférence 12 à 24 atomes de carbone, w représente un entier dans la plage de 0 à 20, de préférence de 0 à 10, plus préférablement de 0 à 6, et encore plus préférablement de 0 à 4, A'O est un groupe alkylénoxy contenant 2 à 4 atomes de carbone, de préférence 2

ou 3 atomes de carbone, plus préférablement 2 atomes de carbone ; T est alkylène avec 1 à 6 atomes de carbone, de préférence 1 à 4 atomes de carbone, de manière préférée entre toutes 2 ou 3 atomes de carbone,
- y est un entier de 0 à 5, de préférence de 0 à 3, plus préférablement y est 0 ou 1, encore plus préférablement y est 0, et
- G représente un groupe de formule (IV) :

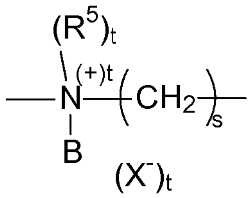

(IV)

dans laquelle $R^5$, X et t sont tels que définis ci-dessus,
- B est choisi parmi un groupe alkyle en $C_1$-$C_4$, aryle ou arylalkyle (par exemple phényle, phénylalkyle, tel que benzyle), et
- s est 1, 2 ou 3, de préférence 2 ou 3,

sachant qu'au moins un des groupes $R^{10}$ représente $R^7$, et au moins un autre des groupes $R^{10}$ représente $R^1$-$(G)_y$- et chaque t est indépendant des autres.

**14.** Composé selon la revendication 13, dans lequel :

- $R^2$ est choisi dans le groupe constitué d'un radical hydrocarbyle divalent ayant de 1 à 10, de préférence de 2 à 6, et de manière préférée entre toutes 4 atomes de carbone, limites incluses,
- lorsque $R^{10}$ est $R^7$, $R^7$ est choisi parmi un groupe hydrocarbyle ayant 1 à 4 atomes de carbone, de préférence 1 à 2 atomes de carbone et de manière préférée entre toutes $R^7$ est méthyle,
- lorsque $R^{10}$ est $R^1$-(G)y-, y = 0 et $R^1$ est choisi parmi un groupe hydrocarbyle ayant 8 à 24 atomes de carbone, de préférence 12 à 24 atomes de carbone,
- QO représente un groupe éthoxy et
- p, $q_1$, $q_2$, $q_3$, $q_4$, t, $R^5$ et X sont tels que définis dans la revendication 13.

**15.** Composé selon la revendication 13 ou la revendication 14, dans lequel tous les "t" sont égaux à 1.

**16.** Composé selon l'une quelconque des revendications 13 à 15, étant obtenu à partir de la ou les réaction(s) de condensation par estérification simultanée(s)/séquentielle(s)/alternée(s) de :

- au moins un composé de formule (I), dans lequel y = 0, et $R^1$ est choisi parmi un groupe hydrocarbyle ayant 8 à 24 atomes de carbone, de préférence 10 à 24, plus préférablement 12 à 24 atomes de carbone,
- au moins un composé de formule (II), et
- au moins un composé de formule (III), dans lequel $R^7$ est un groupe hydrocarbyle ayant 1 à 7, de préférence 1 à 6 atomes de carbone, plus préférablement 1 à 4 atomes de carbone,

ainsi que leurs produits de réaction de quaternarisation partielle ou totale.

**17.** Utilisation d'un composé selon l'une quelconque des revendications 1 à 16 en tant que collecteur pour l'enrichissement (flottation) de minerai, en tant qu'inhibiteur de corrosion, en tant que viscosificateur, émulsifiant ou stabilisant utile pour l'industrie du pétrole et du gaz, en tant que modificateur d'argile, en tant que promoteur d'adhérence, en tant qu'additif antiagglomérant, en tant qu'additif dans des produits de soin capillaire, en tant qu'assouplissant de tissu, en tant qu'agent antistatique dans des polymères, en tant qu'additif d'émulsion de bitume, en tant qu'agent cationique de détergence, en tant qu'additif pour des engrais, en tant qu'antiagglomérant pour des hydrates, en tant qu'additif de lubrification ou de promoteur d'adhérence, et similaire.

## Patentansprüche

**1.** Verbindung, die durch die Kondensation durch Veresterung des Folgenden erhältlich ist:

A) eines alkoxylierten Fettamins der Formel (I) oder des Produkts einer partiellen oder vollständigen Quaternisierung des alkoxylierten Fettamins der Formel (I):

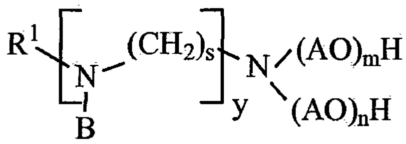

(I),

wobei:

- $R^1$ ausgewählt ist aus einer Kohlenwasserstoffgruppe mit 8 bis 24 Kohlenstoffatomen, vorzugsweise 10 bis 24, stärker bevorzugt 12 bis 24 Kohlenstoffatomen, und einer Gruppe der Formel $R^4$-O-(A'O)$_w$-T-, wobei $R^4$ eine Kohlenwasserstoffgruppe mit 8 bis 24 Kohlenstoffatomen, vorzugsweise 12 bis 24 Kohlenstoffatomen, ist, w eine ganze Zahl im Bereich von 0 bis 20, vorzugsweise von 0 bis 10, stärker bevorzugt von 0 bis 6 und noch mehr bevorzugt von 0 bis 4, darstellt, A'O eine Alkylenoxygruppe ist, die 2 bis 4 Kohlenstoffatome, vorzugsweise 2 oder 3 Kohlenstoffatome, stärker bevorzugt 2 Kohlenstoffatome enthält; T ein Alkylen mit 1 bis 6 Kohlenstoffatomen, vorzugsweise 1 bis 4 Kohlenstoffatomen, am meisten bevorzugt 2 oder 3 Kohlenstoffatomen, ist,
- AO eine Alkylenoxygruppe ist, die 2 bis 4 Kohlenstoffatome, vorzugsweise 2 oder 3 Kohlenstoffatome, stärker bevorzugt 2 Kohlenstoffatome, enthält,
- B aus einer $C_1$-$C_4$-Alkyl- Aryl- oder Arylalkylgruppe (beispielsweise Phenyl, Phenylalkyl, wie Benzyl) ausgewählt ist,
- m eine ganze Zahl zwischen 1 und 20, vorzugsweise zwischen 1 und 10, stärker bevorzugt zwischen 1 und 6 und noch mehr bevorzugt zwischen 1 und 4, darstellt, Grenzwerte eingeschlossen,
- n eine ganze Zahl zwischen 1 und 20, vorzugsweise zwischen 1 und 10, stärker bevorzugt zwischen 1 und 6 und noch mehr bevorzugt zwischen 1 und 4, darstellt, Grenzwerte eingeschlossen,
- s 1, 2 oder 3, vorzugsweise 2 oder 3, ist, und
- y eine ganze Zahl von 0 bis 5, vorzugsweise von 0 bis 3, ist, y stärker bevorzugt 0 oder 1 ist, y noch mehr bevorzugt 0 ist,

B) mit einer Dicarbonsäure oder einem Derivat davon der Formel (II):

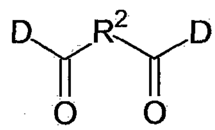

(II),

wobei

- D aus -F, -Cl, -Br und -$OR^3$ ausgewählt ist, wobei $R^3$ Wasserstoff oder eine $C_1$-$C_4$-Alkylgruppe ist,
- $R^2$ ausgewählt ist aus der Gruppe bestehend aus:
    - ◦einer direkten Bindung,
    - ◦einer gesättigten oder ungesättigten, linearen oder verzweigten $C_1$-$C_{20}$-Kohlenwasserstoffkette, die gegebenenfalls durch eine oder mehrere -OH-Gruppen substituiert ist, vorzugsweise einem Alkylenrest der Formel -$(CH_2)_z$-, wobei z eine ganze Zahl von 1 bis 20, vorzugsweise von 1 bis 10, vorzugsweise von 2 bis 6 und am meisten bevorzugt 4, ist, einem substituierten Alkylenrest, wobei der Alkylenrest durch 1 oder 2 -OH-Gruppen substituiert ist, einem Alkenylenrest mit 1 bis 20, vorzugsweise 1 bis 10 Kohlenstoffatomen, einem substituierten Alkenylenrest, wobei der Alkenylenrest durch 1 oder 2 Methyl- und/oder Methylengruppen substituiert ist,
    - ◦ einer Cycloalkylengruppe,
    - ◦ Cycloalkenylen und
    - ◦ Arylen

C) mit einem (Alkyl)Alkanolaminderivat der Formel (III) oder dem Produkt einer partiellen oder vollständigen

Quaternisierung des (Alkyl)Alkanolaminderivats der Formel (III):

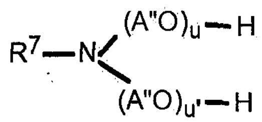

(III),

wobei:

- A"O eine Alkylenoxygruppe darstellt, die 2 bis 4 Kohlenstoffatome, vorzugsweise 2 oder 3 Kohlenstoffatome, stärker bevorzugt 2 Kohlenstoffatome enthält,
- u eine ganze Zahl zwischen 1 und 20, vorzugsweise zwischen 1 und 10, stärker bevorzugt zwischen 1 und 6 und noch mehr bevorzugt zwischen 1 und 4, darstellt, Grenzwerte eingeschlossen,
- u' eine ganze Zahl zwischen 1 und 20, vorzugsweise zwischen 1 und 10, stärker bevorzugt zwischen 1 und 6 und noch mehr bevorzugt zwischen 1 und 4, darstellt, Grenzwerte eingeschlossen,
- $R^7$ ausgewählt ist aus einer Kohlenwasserstoffgruppe mit 1 bis 7, vorzugsweise 1 bis 6 Kohlenstoffatomen, stärker bevorzugt 1 bis 4 Kohlenstoffatomen, einer Aryl- oder Arylalkylgruppe (beispielsweise einer Phenyl- oder Naphthylgruppe), einer Gruppe der Formel $H-(OA")_v-$ (wobei v eine ganze Zahl zwischen 1 und 20, vorzugsweise zwischen 1 und 10, stärker bevorzugt zwischen 1 und 6 und noch mehr bevorzugt zwischen 1 und 4 darstellt, Grenzwerte eingeschlossen), $HO(CH_2)_q-$ und einer Gruppe der Formel (IV):

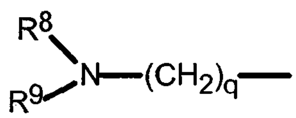

(IV),

wobei $R^8$ und $R^9$, die identisch oder verschieden sein können, aus einer Kohlenwasserstoffgruppe mit 1 bis 6 Kohlenstoffatomen, vorzugsweise 1 bis 4 Kohlenstoffatomen, ausgewählt sind, und q eine ganze Zahl von 1 bis 10, vorzugsweise 2 bis 6, ist, Grenzwerte eingeschlossen, und wobei q am meisten bevorzugt 2 oder 3 ist,
oder $R^8$ und $R^9$ zusammen mit dem Stickstoffatom, mit dem sie verbunden sind, einen Ring mit 5, 6 oder 7 Atomen bilden, der gegebenenfalls ein oder mehrere Heteroatome umfasst, das bzw. die aus Sauerstoff, Stickstoff oder Schwefel ausgewählt ist bzw. sind.

2. Verbindung nach Anspruch 1, wobei der Rest $R^1$ des Fettamins der Formel (I) 8 oder mehr als 8 Kohlenstoffatome, typischerweise von 8 bis 24 Kohlenstoffatome, vorzugsweise von 10 bis 24, stärker bevorzugt von 12 bis 24 Kohlenstoffatome umfasst, Grenzwerte eingeschlossen, und der Rest $R^7$ des (Alkyl)Alkanolaminderivats der Formel (III) 6 Kohlenstoffatome oder weniger, typischerweise von 1 bis 6 Kohlenstoffatome, stärker bevorzugt von 1 bis 4 Kohlenstoffatome umfasst, Grenzwerte eingeschlossen.

3. Verbindung nach Anspruch 1 oder Anspruch 2, wobei der Rest $R^1$ bzw. der Rest $R^7$ des Fettamins der Formel (I) bzw. des (Alkyl)Alkanolaminderivats der Formel (III) derart sind, dass der Unterschied der Zahl von Kohlenstoffatomen, die sie umfassen, größer als 2, typischerweise von 2 bis 23, vorzugsweise von 5 bis 23, stärker bevorzugt von 10 bis 23, beträgt, Grenzwerte eingeschlossen.

4. Verbindung nach einem der Ansprüche 1 bis 3, wobei ein Teil oder die Gesamtheit der Stickstoffatome außerdem mit einem Reagens der Formel $R^5X$ reagiert, wobei $R^5$ aus einer $C_1$-$C_6$-Kohlenwasserstoffgruppe, vorzugsweise einer $C_1$-$C_4$-Kohlenwasserstoff-, Phenyl- und Phenylalkylgruppe, wie Benzyl, ausgewählt ist und X aus Halogenen, Sulfaten, Carbonaten und dergleichen ausgewählt ist.

5. Verbindung nach einem der Ansprüche 1 bis 4, wobei das alkoxylierte Fettamin der Formel (I) die Formel (IA) :

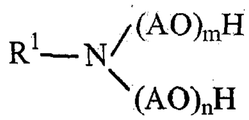

(IA)

aufweist, bei der es sich um das alkoxylierte Fettamin der Formel (I) handelt, und wobei y 0 darstellt und $R^1$, AO, m und n wie in Anspruch 1 definiert sind,
sowie deren entsprechende teilweise oder vollständig quaternisierte Derivate.

**6.** Verbindung nach einem der Ansprüche 1 bis 5, wobei das (Alkyl)Alkanolaminderivat der Formel (III) die Formel (IIIA):

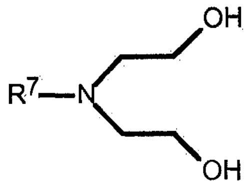

(IIIA)

aufweist, bei der es sich um das (Alkyl)Alkanolamin der Formel (III) handelt, wobei u und u' jeweils 1 darstellen, A"O Ethylenoxy ist und $R^7$ wie in Anspruch 1 definiert ist und vorzugsweise eine Kohlenwasserstoffgruppe mit 1 bis 4 Kohlenstoffatomen ist,
sowie deren entsprechende teilweise oder vollständig quaternisierte Derivate.

**7.** Verbindung nach einem der Ansprüche 1 bis 6, wobei die (Alkyl)Alkanolaminderivate der Formel (III) Triethanolamin, Methyldiethanolamin, Ethyldiethanolamin, Propyldiethanolamin, Butyldiethanolamin, Isobutyldiethanolamin, Pentyldiethanolamin, Phenyldiethanolamin, Hexyldiethanolamin, Heptyldiethanolamin sowie deren entsprechende Alkoxylierungsprodukte umfassen, ohne darauf beschränkt zu sein.

**8.** Verbindung nach einem der Ansprüche 1 bis 7, wobei das Dicarbonsäurederivat der allgemeinen Formel (II) aus einer Dicarbonsäure, einem Dicarbonsäurehalogenid, einem Dicarbonsäurediester oder einem cyclischen Anhydrid einer Dicarbonsäure ausgewählt ist.

**9.** Verbindung nach einem der Ansprüche 1 bis 8, wobei veranschaulichende Beispiele von Dicarbonsäurederivaten der allgemeinen Formel (II) Oxalsäure, Malonsäure, Bernsteinsäure, Glutarsäure, Glutaconsäure, Adipinsäure, Muconsäure, Pimelinsäure, Phthalsäure und deren Isomere, Tetrahydrophthalsäure, Äpfelsäure, Maleinsäure, Fumarsäure, Korksäure, Mesaconsäure, Sebacinsäure, Azelainsäure, Weinsäure, Itaconsäure, Glutinsäure, Citraconsäure, Brassylsäure, Dodecandisäure, Traumatinsäure, Thapsiasäure, deren entsprechende Säurechloride, deren entsprechende Methyl- oder Ethylester und deren entsprechende cyclische Anhydride sowie Mischungen davon umfassen.

**10.** Verbindung nach einem der Ansprüche 1 bis 9, wobei das Stoffmengenverhältnis zwischen den Reagenzien [(I) + (III)] und (II) von 2:1 bis 1:2, vorzugsweise von 1,5:1 bis 1:1,5 und am meisten bevorzugt von 1,4:1 bis 1:1,4, beträgt.

**11.** Verbindung nach einem der Ansprüche 1 bis 9, wobei das Stoffmengenverhältnis zwischen den Reagenzien [(I) + (III)] und (II) von 2:1 bis 1:1, vorzugsweise von 2:1 bis 1,2:1, am meisten bevorzugt von 2:1 bis 1,3:1, beträgt.

**12.** Verbindung nach einem der Ansprüche 1 bis 11, wobei das Stoffmengenverhältnis zwischen (I) und (III) von 15:1 bis 1:15, vorzugsweise von 10:1 bis 1:10, stärker bevorzugt von 4:1 bis 1:4 und am meisten bevorzugt von 2:1 bis 1:2 beträgt.

**13.** Verbindung der allgemeinen Formel (1):

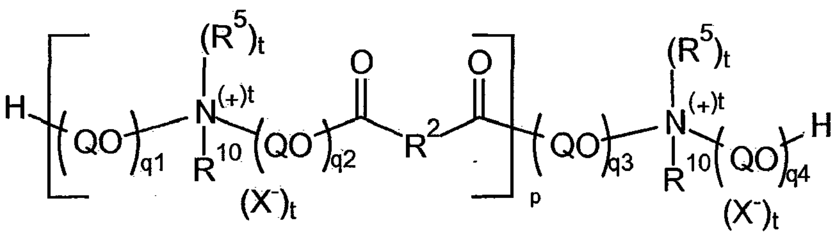

(1) ,

wobei:

- $R^2$ ausgewählt ist aus der Gruppe bestehend aus:

  ◦ einer direkten Bindung,
  ◦ einer gesättigten oder ungesättigten, linearen oder verzweigten $C_1$-$C_{20}$-Kohlenwasserstoffkette, die gegebenenfalls durch eine oder mehrere -OH-Gruppen substituiert ist, vorzugsweise einem Alkylenrest der Formel $-(CH_2)_z-$, wobei z eine ganze Zahl von 1 bis 20, vorzugsweise von 1 bis 10, vorzugsweise von 2 bis 6 und am meisten bevorzugt 4, ist, einem substituierten Alkylenrest, wobei der Alkylenrest durch 1 oder 2 -OH-Gruppen substituiert ist, einem Alkenylenrest mit 1 bis 20, vorzugsweise 1 bis 10 Kohlenstoffatomen, einem substituierten Alkenylenrest, wobei der Alkenylenrest durch 1 oder 2 Methyl- und/oder Methylengruppen substituiert ist,
  ◦ einer Cycloalkylengruppe,
  ◦ Cycloalkenylen und
  ◦ Arylen

- $R^5$ aus einer $C_1$-$C_6$-Kohlenwasserstoffgruppe, vorzugsweise einer $C_1$-$C_4$-Alkyl-, Phenyl- und Phenylalkylgruppe, wie Benzyl, ausgewählt ist,
- X aus Halogenen, Sulfaten, Carbonaten und dergleichen ausgewählt ist,
- t 0 oder 1 ist
- p eine ganze Zahl im Bereich von 1 bis 15, vorzugsweise von 1 bis 10, stärker bevorzugt von 1 bis 5, ist, Grenzwerte eingeschlossen,
- QO eine Alkylenoxygruppe darstellt, die 2 bis 4 Kohlenstoffatome, vorzugsweise 2 oder 3 Kohlenstoffatome, stärker bevorzugt 2 Kohlenstoffatome, enthält, wobei alle in der Verbindung der Formel (1) vorhandenen Q identisch oder verschieden sein können,
- $q_1$, $q_2$, $q_3$, $q_4$, die identisch oder verschieden voneinander sein können, jeweils eine ganze Zahl zwischen 1 und 20, vorzugsweise zwischen 1 und 10, stärker bevorzugt zwischen 1 und 6 und noch mehr bevorzugt zwischen 1 und 4, darstellen, Grenzwerte eingeschlossen,
- jede Gruppe $R^{10}$ unabhängig von den anderen $R^7$ oder eine $R^1$-$(G)_y$--Gruppe darstellt,
- $R^7$ ausgewählt ist aus einer Kohlenwasserstoffgruppe mit 1 bis 7, vorzugsweise 1 bis 6 Kohlenstoffatomen, stärker bevorzugt 1 bis 4 Kohlenstoffatomen, einer Aryl- oder Arylalkylgruppe (beispielsweise einer Phenyl- oder Naphthylgruppe), einer Gruppe der Formel $H$-$(OA'')_v$- (wobei v eine ganze Zahl zwischen 1 und 20, vorzugsweise zwischen 1 und 10, stärker bevorzugt zwischen 1 und 6 und noch mehr bevorzugt zwischen 1 und 4 darstellt, Grenzwerte eingeschlossen), $HO(CH_2)_q$- und einer Gruppe der Formel (IV):

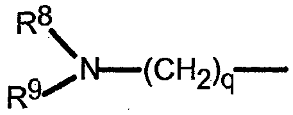

(IV),

wobei $R^8$ und $R^9$, die identisch oder verschieden sein können, aus einer Kohlenwasserstoffgruppe mit 1 bis 6 Kohlenstoffatomen, vorzugsweise 1 bis 4 Kohlenstoffatomen, ausgewählt sind, und q eine ganze Zahl von 1 bis 10, vorzugsweise 2 bis 6, ist, Grenzwerte eingeschlossen, und wobei q am meisten bevorzugt 2 oder 3 ist,
- oder $R^8$ und $R^9$ zusammen mit dem Stickstoffatom, mit dem sie verbunden sind, einen Ring mit 5, 6 oder 7 Atomen bilden, der gegebenenfalls ein oder mehrere Heteroatome umfasst, das bzw. die aus Sauerstoff, Stickstoff oder Schwefel ausgewählt ist bzw. sind,
- $R^1$ ausgewählt ist aus einer Kohlenwasserstoffgruppe mit 8 bis 24 Kohlenstoffatomen, vorzugsweise 10 bis

24, stärker bevorzugt 12 bis 24 Kohlenstoffatomen, und einer Gruppe der Formel $R^4$-O-(A'O)$_w$-T-, wobei $R^4$ eine Kohlenwasserstoffgruppe mit 8 bis 24 Kohlenstoffatomen, vorzugsweise 12 bis 24 Kohlenstoffatomen, ist, w eine ganze Zahl im Bereich von 0 bis 20, vorzugsweise von 0 bis 10, stärker bevorzugt von 0 bis 6 und noch mehr bevorzugt von 0 bis 4, darstellt, A'O eine Alkylenoxygruppe ist, die 2 bis 4 Kohlenstoffatome, vorzugsweise 2 oder 3 Kohlenstoffatome, stärker bevorzugt 2 Kohlenstoffatome enthält; T ein Alkylen mit 1 bis 6 Kohlenstoffatomen, vorzugsweise 1 bis 4 Kohlenstoffatomen, am meisten bevorzugt 2 oder 3 Kohlenstoffatomen, ist,
- y eine ganze Zahl von 0 bis 5, vorzugsweise von 0 bis 3, ist, y stärker bevorzugt 0 oder 1 ist, y noch mehr bevorzugt 0 ist, und
- G eine Gruppe der Formel (IV):

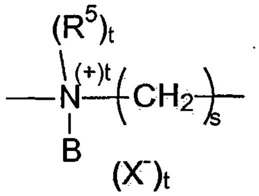

(IV)

darstellt, wobei $R^5$, X und t wie oben definiert sind,

- B aus einer $C_1$-$C_4$-Alkyl- Aryl- oder Arylalkylgruppe (beispielsweise Phenyl, Phenylalkyl, wie Benzyl) ausgewählt ist und
- s 1, 2 oder 3, vorzugsweise 2 oder 3, ist,

wobei mindestens eine der $R^{10}$-Gruppen $R^7$ darstellt und mindestens eine andere der $R^{10}$-Gruppen $R^1$- (G)$_y$-darstellt und jedes t unabhängig von den anderen ist.

**14.** Verbindung nach Anspruch 13, wobei:

- $R^2$ ausgewählt ist aus der Gruppe bestehend aus einem zweiwertigen Kohlenwasserstoffrest mit 1 bis 10, vorzugsweise 2 bis 6 und am meisten bevorzugt 4 Kohlenstoffatomen, Grenzwerte eingeschlossen,
- wenn $R^{10}$ $R^7$ ist, $R^7$ aus einer Kohlenwasserstoffgruppe mit 1 bis 4 Kohlenstoffatomen, vorzugsweise 1 bis 2 Kohlenstoffatomen ausgewählt ist und am meisten bevorzugt $R^7$ Methyl ist,
- wenn $R^{10}$ $R^1$-(G)$_y$- ist, y = 0 und $R^1$ aus einer Kohlenwasserstoffgruppe mit 8 bis 24 Kohlenstoffatomen, vorzugsweise 12 bis 24 Kohlenstoffatomen, ausgewählt ist,
- QO eine Ethoxygruppe darstellt und
- p, $q_2$, $q_2$, $q_3$, $q_2$, t, $R^5$ und X wie in Anspruch 13 definiert sind.

**15.** Verbindung nach Anspruch 13 oder Anspruch 14, wobei alle "t" gleich 1 sind.

**16.** Verbindung nach einem der Ansprüche 13 bis 15, die durch die gleichzeitige(n) / aufeinanderfolgende(n) / abwechselnde(n) Kondensationsreaktion(en) durch Veresterung von Folgendem erhalten wird:

- mindestens einer Verbindung der Formel (I), wobei y = 0 und $R^1$ aus einer Kohlenwasserstoffgruppe mit 8 bis 24 Kohlenstoffatomen, vorzugsweise 10 bis 24, stärker bevorzugt 12 bis 24 Kohlenstoffatomen, ausgewählt ist,
- mindestens einer Verbindung der Formel (II) und
- mindestens einer Verbindung der Formel (III), wobei $R^7$ eine Kohlenwasserstoffgruppe mit 1 bis 7, vorzugsweise 1 bis 6 Kohlenstoffatomen, stärker bevorzugt 1 bis 4 Kohlenstoffatomen, ist,

sowie den Produkten einer partiellen oder vollständigen Quaternisierungsreaktion davon.

**17.** Verwendung einer Verbindung nach einem der Ansprüche 1 bis 16 als Abscheider zur Anreicherung (Flotation) von Erzen, als Korrosionsinhibitor, als viskositätsverbesserndes Mittel, Emulgator oder Stabilisator, das bzw. der in der Erdöl- und Gasindustrie brauchbar ist, als Tonmodifizierungsmittel, als Haftungsvermittler, als Antiagglomerationsadditiv, als Additiv in Haarpflegeprodukten, als Gewebeweichspülmittel, als Antistatikum in Polymeren, als Additiv zum Emulgieren von Bitumen, als kationisch machendes Mittel für Detergenzien, als Düngemitteladditiv, als Agglomerationsinhibitor für Hydrate, als gleitfähig machendes oder haftvermittendes Additiv und dergleichen.

## Claims

1. Compound which may be obtained by esterification condensation of:

A/ an alkoxylated fatty amine of formula (I), or the product of partial or total quaternization of said alkoxylated fatty amine of formula (I):

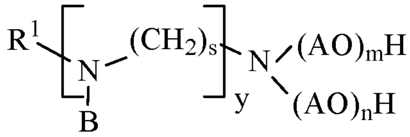

(I)

in which:

$R^1$ is chosen from a hydrocarbyl group containing 8 to 24 carbon atoms, preferably 10 to 24 and more preferably 12 to 24 carbon atoms, and a group of formula $R^4$-O-(A'O)$_w$-T-, in which $R^4$ is a hydrocarbyl group containing 8 to 24 carbon atoms and preferably 12 to 24 carbon atoms, w represents an integer in the range from 0 to 20, preferably from 0 to 10, more preferably from 0 to 6 and even more preferably from 0 to 4, A'O is an alkylenoxy group containing 2 to 4 carbon atoms, preferably 2 or 3 carbon atoms, more preferably 2 carbon atoms; T is alkylene with 1 to 6 carbon atoms,
preferably from 1 to 4 carbon atoms and most preferably 2 or 3 carbon atoms,
AO is an alkylenoxy group containing 2 to 4 carbon atoms, preferably 2 or 3 carbon atoms, more preferably 2 carbon atoms,
B is chosen from a $C_1$-$C_4$ alkyl, an aryl and an arylalkyl (for example phenyl or phenylalkyl, such as benzyl) group,
m represents an integer between 1 and 20, preferably between 1 and 10, more preferably between 1 and 6, and even more preferably between 1 and 4, limits inclusive,
n represents an integer between 1 and 20, preferably between 1 and 10, more preferably between 1 and 6, and even more preferably between 1 and 4, limits inclusive,
s is 1, 2 or 3, preferably 2 or 3, and
y is an integer from 0 to 5, preferably from 0 to 3, more preferably y is 0 or 1, even more preferably y is 0,

B/ with a dicarboxylic acid, or a derivative thereof, of formula (II):

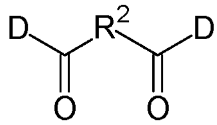

(II),

in which

D is chosen from -F, -Cl, Br and -OR$^3$, in which $R^3$ is hydrogen or a $C_1$-$C_4$ alkyl group,
$R^2$ is chosen from the group constituted by:

a direct bond,
a linear or branched, saturated or unsaturated $C_1$-$C_{20}$ hydrocarbon-based chain optionally substituted with one or more -OH groups, preferably an alkylene radical of formula $-(CH_2)_z-$, in which z is an integer from 1 to 20, preferably from 1 to 10, preferably from 2 to 6 and most preferably equal to 4, a substituted alkylene radical, said alkylene radical being substituted with 1 or 2-OH groups, an alkenylene radical containing from 1 to 20 and preferably from 1 to 10 carbon atoms, a substituted alkenylene radical, said alkenylene radical being substituted with 1 or 2 methyl and/or methylene groups,
a cycloalkylene group,
cycloalkenylene and
arylene

C/ with an (alkyl)alkanolamine of formula (III) derivative or product of partial or total quaternization of said (alkyl)alkanolamine derivative of formula (III):

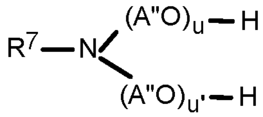

(III)

in which:

A"0 represents an alkylenoxy group containing from 2 to 4 carbon atoms, preferably 2 or 3 carbon atoms, more preferably 2 carbon atoms,
u represents an integer between 1 and 20, preferably between 1 and 10, more preferably between 1 and 6, and even more preferably between 1 and 4, limits inclusive,
u' represents an integer between 1 and 20, preferably between 1 and 10, more preferably between 1 and 6, and even more preferably between 1 and 4, limits inclusive,
$R^7$ is chosen from a hydrocarbyl group containing 1 to 7, preferably 1 to 6 carbon atoms, more preferably 1 to 4 carbon atoms, an aryl or arylalkyl group (for example, a phenyl or naphthyl group), a group of formula $H\text{-}(OA")_v\text{-}$ (in which v represents an integer between 1 and 20, preferably between 1 and 10, more preferably between 1 and 6, and even more preferably between 1 and 4, limits inclusive), $HO(CH_2)_q\text{-}$, and a group of formula (IV):

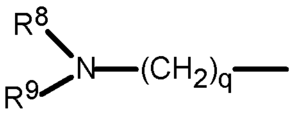

(IV),

in which $R^8$ and $R^9$, which may be identical or different, are chosen from a hydrocarbyl group containing 1 to 6 carbon atoms, preferably 1 to 4 carbon atoms, and q is an integer from 1 to 10, preferably from 2 to 6, limits inclusive, and most preferably q is 2 or 3,
or $R^8$ and $R^9$, together with the nitrogen atom to which they are attached, form a 5-, 6- or 7-membered ring, optionally including one or more heteroatoms chosen from oxygen, nitrogen and sulfur.

**2.** Compound according to Claim 1, in which the radical $R^1$ of the fatty amine of formula (I) includes 8 or more than 8 carbon atoms, typically from 8 to 24 carbon atoms, preferably from 10 to 24, more preferably from 12 to 24 carbon atoms, limits inclusive, and the radical $R^7$ of the (alkyl)alkanolamine derivative of formula (III) includes 6 carbon atoms or less, typically from 1 to 6 carbon atoms, more preferably 1 to 4 carbon atoms, limits inclusive.

**3.** Compound according to Claim 1 or Claim 2, in which the radical $R^1$ and the radical $R^7$ of the fatty amine of formula (I) and of the (alkyl)alkanolamine derivative of formula (III), respectively, are such that the difference in the number of carbon atoms they include is greater than 2, typically from 2 to 23, preferably from 5 to 23, more preferably from 10 to 23, limits inclusive.

**4.** Compound according to any one of Claims 1 to 3, in which some or all of the nitrogen atoms also react with a reagent of formula $R^5X$, in which $R^5$ is chosen from a $C_1\text{-}C_6$ hydrocarbyl group, preferably a $C_1\text{-}C_4$ alkyl, a phenyl and a phenylalkyl, such as benzyl, group, and X is chosen from halogens, sulfates, carbonates, and the like.

**5.** Compound according to any one of Claims 1 to 4, in which the alkoxylated fatty amine of formula (I) is of formula (IA):

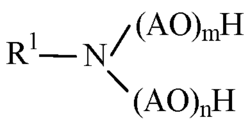

(IA)

which is the alkoxylated fatty amine of formula (I) in which y represents 0 and $R^1$, AO, m and n are as defined

in Claim 1,
and also the corresponding partially or totally quaternized derivatives thereof.

**6.** Compound according to any one of Claims 1 to 5, in which the (alkyl)alkanolamine derivative of formula (III) is of formula (IIIA):

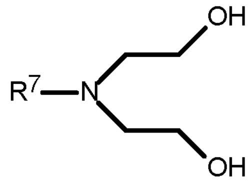

(IIIA)

which is the (alkyl)alkanolamine of formula (III) in which u and u' each represent 1, A"O is ethylenoxy and $R^7$ is as defined in Claim 1, and is preferably a hydrocarbyl group containing 1 to 4 carbon atoms,
and also the corresponding partially or totally quaternized derivatives thereof.

**7.** Compound according to any one of Claims 1 to 6, in which the (alkyl)alkanolamine derivatives of formula (III) comprise, but are not limited to, triethanolamine, methyldiethanolamine, ethyldiethanolamine, propyldiethanolamine, butyldiethanolamine, isobutyldiethanolamine, pentyldiethanolamine, phenyldiethanolamine, hexyldiethanolamine, heptyldiethanolamine, and also the corresponding alkoxylation products thereof.

**8.** Compound according to any one of Claims 1 to 7, in which the dicarboxylic acid derivative of general formula (II) is chosen from a dicarboxylic acid, a dicarboxylic acid halide, a dicarboxylic acid diester or a cyclic anhydride of a dicarboxylic acid.

**9.** Compound according to any one of Claims 1 to 8, in which illustrative examples of dicarboxylic acid derivatives of general formula (II) comprise oxalic acid, malonic acid, succinic acid, glutaric acid, glutaconic acid, adipic acid, muconic acid, pimelic acid, phthalic acid and isomers thereof, tetrahydrophthalic acid, malic acid, maleic acid, fumaric acid, suberic acid, mesaconic acid, sebacic acid, azelaic acid, tartaric acid, itaconic acid, glutinic acid, citraconic acid, brassylic acid, dodecanedioic acid, traumatic acid, thapsic acid, the corresponding acid chlorides thereof, the corresponding methyl or ethyl esters thereof, and the corresponding cyclic anhydrides thereof, and also mixtures thereof.

**10.** Compound according to any one of Claims 1 to 9, in which the mole ratio between the reagents [(I) + (III)] and (II) is from 2:1 to 1:2, preferably from 1.5:1 to 1:1.5 and most preferably from 1.4:1 to 1:1.4.

**11.** Compound according to any one of Claims 1 to 9, in which the mole ratio between the reagents [(I) + (III)] and (II) is from 2:1 to 1:1, preferably from 2:1 to 1.2:1 and most preferably from 2:1 to 1.3:1.

**12.** Compound according to any one of Claims 1 to 11, in which the mole ratio between (I) and (III) is from 15:1 to 1:15, preferably from 10:1 to 1:10, more preferably from 4:1 to 1:4 and most preferably from 2:1 to 1:2.

**13.** Compound of general formula (1):

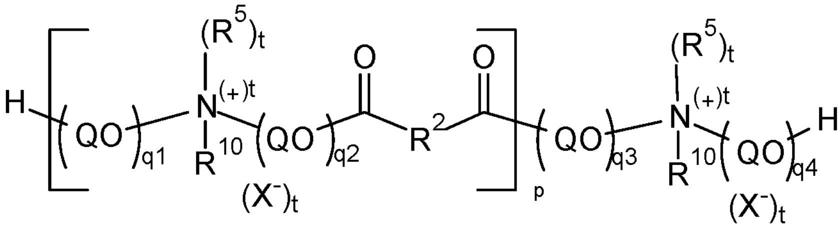

(1)

in which:
$R^2$ is chosen from the group constituted by:

a direct bond,

a linear or branched, saturated or unsaturated $C_1$-$C_{20}$ hydrocarbon-based chain optionally substituted with one or more -OH groups, preferably an alkylene radical of formula $-(CH_2)_z-$, in which z is an integer from 1 to 20, preferably from 1 to 10, preferably from 2 to 6 and most preferably equal to 4, a substituted alkylene radical, said alkylene radical being substituted with 1 or 2 - OH groups, an alkenylene radical containing from 1 to 20 and preferably from 1 to 10 carbon atoms, a substituted alkenylene radical, said alkenylene radical being substituted with 1 or 2 methyl and/or methylene groups,

a cycloalkylene group,

cycloalkenylene and

arylene

$R^5$ is chosen from a $C_1$-$C_6$ hydrocarbyl group, preferably a $C_1$-$C_4$ alkyl group, a phenyl group and a phenylalkyl group, such as benzyl,

X is chosen from halogens, sulfates, carbonates, and the like,

t is 0 or 1,

p is an integer in the range from 1 to 15, preferably from 1 to 10, more preferably from 1 to 5, limits inclusive,

QO represents an alkylenoxy group containing from 2 to 4 carbon atoms, preferably 2 or 3 carbon atoms, more preferably 2 carbon atoms, given that all the Q groups present in the compound of formula (1) may be identical or different, $q_1$, $q_2$, $q_3$ and $q_4$, which may be identical to or different from each other, each represent an integer between 1 and 20, preferably between 1 and 10, more preferably between 1 and 6 and even more preferably between 1 and 4, limits inclusive,

each group $R^{10}$, independently of each other, represents $R^7$ or a group $R^7$-$(G)_y$-,

$R^7$ is chosen from a hydrocarbyl group containing 1 to 7, preferably 1 to 6 carbon atoms, more preferably 1 to 4 carbon atoms, an aryl or arylalkyl group (for example, a phenyl or naphthyl group), a group of formula $H-(OA")_v-$ (in which v represents an integer between 1 and 20, preferably between 1 and 10, more preferably between 1 and 6, and even more preferably between 1 and 4, limits inclusive), $HO(CH_2)_q-$, and a group of formula (IV):

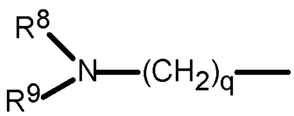

(IV),

in which $R^8$ and $R^9$, which may be identical or different, are chosen from a hydrocarbyl group containing 1 to 6 carbon atoms, preferably 1 to 4 carbon atoms, and q is an integer from 1 to 10, preferably from 2 to 6, limits inclusive, and most preferably q is 2 or 3,

or $R^8$ and $R^9$, together with the nitrogen atom to which they are attached, form a 5-, 6- or 7-membered ring, optionally including one or more heteroatoms chosen from oxygen, nitrogen and sulfur,

$R^1$ is chosen from a hydrocarbyl group containing 8 to 24 carbon atoms, preferably 10 to 24 and more preferably 12 to 24 carbon atoms, and a group of formula $R^4$-O-$(A'O)_w$-T-, in which $R^4$ is a hydrocarbyl group containing 8 to 24 carbon atoms and preferably 12 to 24 carbon atoms, w represents an integer in the range from 0 to 20, preferably from 0 to 10, more preferably from 0 to 6 and even more preferably from 0 to 4, A'O is an alkylenoxy group containing 2 to 4 carbon atoms, preferably 2 or 3 carbon atoms, more preferably 2 carbon atoms; T is alkylene with 1 to 6 carbon atoms, preferably 1 to 4 carbon atoms and most preferably 2 or 3 carbon atoms,

y is an integer from 0 to 5, preferably from 0 to 3, more preferably y is 0 or 1, even more preferably y is 0, and

G represents a group of formula (IV):

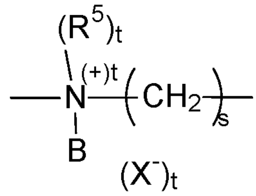

(IV)

in which $R^5$, X and t are as defined above,

B is chosen from a $C_1$-$C_4$ alkyl, an aryl and an arylalkyl (for example phenyl or phenylalkyl, such as benzyl) group, and

s is 1, 2 or 3, preferably 2 or 3,

given that at least one of the groups $R^{10}$ represents $R^7$, and at least one other of the groups $R^{10}$ represents $R^1$-$(G)_y$- and each t is independent of the others.

**14.** Compound according to Claim 13, in which:

$R^2$ is chosen from the group consisting of a divalent hydrocarbyl radical containing from 1 to 10, preferably from 2 to 6 and most preferably 4 carbon atoms, limits inclusive,
when $R^{10}$ is $R^7$, $R^7$ is chosen from a hydrocarbyl group containing 1 to 4 carbon atoms, preferably 1 to 2 carbon atoms and most preferably $R^7$ is methyl,
when $R^{10}$ is $R^1$-(G)y-, y = 0 and $R^1$ is chosen from a hydrocarbyl group containing 8 to 24 carbon atoms, preferably 12 to 24 carbon atoms,
QO represents an ethoxy group and
p, $q_1$, $q_2$, $q_3$, $q_4$, t, $R^5$ and X are as defined in Claim 13.

**15.** Compound according to Claim 13 or Claim 14, in which all the "t" are equal to 1.

**16.** Compound according to any one of Claims 13 to 15, which is obtained from simultaneous/sequential/alternating esterification condensation reaction(s) of:

at least one compound of formula (I), in which y = 0 and $R^1$ is chosen from a hydrocarbyl group containing 8 to 24 carbon atoms, preferably 10 to 24 and more preferably 12 to 24 carbon atoms,
at least one compound of formula (II), and
at least one compound of formula (III), in which $R^7$ is a hydrocarbyl group containing 1 to 7 and preferably 1 to 6 carbon atoms, more preferably 1 to 4 carbon atoms,
and also the products of partial or total quaternization reaction thereof.

**17.** Use of a compound according to any one of Claims 1 to 16 as a collector for ore enrichment (flotation), as a corrosion inhibitor, as a viscosity enhancer, emulsifier or stabilizer that is useful for the oil and gas industry, as a clay modifier, as an adhesion promoter, as an antiagglomerant additive, as an additive in haircare products, as a fabric softener, as an antistatic agent in polymers, as a bitumen emulsion additive, as a detergency cationic agent, as a fertilizer additive, as an antiagglomerant for hydrates, as a lubrication or adhesion-promoting additive, and the like.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 0035263 A
- EP 0144975 A
- WO 2008089906 A
- JP 8291281 A
- WO 2011147855 A